(19) Europäisches Patentamt
European Patent Office
Office européen des brevets

(11) **EP 3 310 432 B1**

(12) **EUROPÄISCHE PATENTSCHRIFT**

(45) Veröffentlichungstag und Bekanntmachung des Hinweises auf die Patenterteilung:
**01.01.2020   Patentblatt 2020/01**

(21) Anmeldenummer: **16732270.0**

(22) Anmeldetag: **22.06.2016**

(51) Int Cl.:
*A61N 1/36* (2006.01)          *A61B 5/00* (2006.01)
*A61N 2/02* (2006.01)          *A61B 5/053* (2006.01)
*A61B 5/11* (2006.01)          *A61N 2/00* (2006.01)
*A61B 5/0476* (2006.01)       *A61B 5/0488* (2006.01)
*A61M 21/00* (2006.01)       *A61M 21/02* (2006.01)

(86) Internationale Anmeldenummer:
**PCT/EP2016/064472**

(87) Internationale Veröffentlichungsnummer:
**WO 2016/207247 (29.12.2016 Gazette 2016/52)**

(54) **VORRICHTUNG ZUR EFFEKTIVEN NICHT-INVASIVEN ZWEI-STUFEN-NEUROSTIMULATION**

DEVICE FOR EFFECTIVE NON-INVASIVE TWO-STAGE NEUROSTIMULATION

DISPOSITIF POUR NEUROSTIMULATION EFFICACE NON INVASIVE EN DEUX ÉTAPES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.06.2015   DE 102015109986**

(43) Veröffentlichungstag der Anmeldung:
**25.04.2018   Patentblatt 2018/17**

(73) Patentinhaber: **Forschungszentrum Jülich GmbH 52425 Jülich (DE)**

(72) Erfinder:
• **TASS, Peter Alexander**
  **83684 Tegernsee (DE)**
• **ZEITLER, Magteld**
  **6581 JL Malden (NL)**

(74) Vertreter: **Müller-Boré & Partner Patentanwälte PartG mbB Friedenheimer Brücke 21 80639 München (DE)**

(56) Entgegenhaltungen:
**DE-A1-102008 052 078     DE-A1-102009 015 723
DE-A1-102010 016 404     DE-A1-102010 016 461**

EP 3 310 432 B1

**Beschreibung**

Die Erfindung betrifft eine Vorrichtung zur effektiven nicht-invasiven Zwei-Stufen-Neurostimulation mittels variierender Reizsequenzen.

Bei Patienten mit neurologischen oder psychiatrischen Erkrankungen, z. B. Morbus Parkinson, essentiellem Tremor, Dystonie, Funktionsstörungen nach Schlaganfall, Migräne, Zwangserkrankungen, Epilepsie, Tinnitus, Schizophrenie, Depression, Borderline Persönlichkeitsstörung sowie Reizdarmsyndrom, sind Nervenzellverbände in umschriebenen Bereichen des Gehirns krankhaft synchroner Weise aktiv. In diesem Fall bildet eine große Anzahl von Neuronen synchron Aktionspotentiale aus, d. h., die beteiligten Neuronen feuern übermäßig synchron. Beim Gesunden hingegen feuern die Neuronen in diesen Hirngebieten qualitativ anders, z. B. auf unkorrelierte Weise.

Beim Morbus Parkinson verändert die pathologisch synchrone Aktivität im Thalamus und in den Basalganglien die neuronale Aktivität in anderen Hirngebieten, z. B. in Arealen der Großhirnrinde wie dem primär motorischen Cortex. Dabei zwingt die pathologisch synchrone Aktivität im Bereich des Thalamus und der Basalganglien beispielsweise den Großhirnrindenarealen ihren Rhythmus auf, so dass schließlich die von diesen Arealen gesteuerten Muskeln pathologische Aktivität, z. B. ein rhythmisches Zittern (Tremor), entfalten. Beim chronisch subjektivem Tinnitus findet sich krankhafte synchrone Aktivität in einem Netzwerk von auditorischen sowie nicht-auditorischen Hirnarealen.

Bei Patienten mit Hirnerkrankungen und Rückenmarkserkrankungen, welche durch übermäßig synchronisierte neuronale Aktivität gekennzeichnet sind, werden nicht-invasiv bestimmte raum-zeitliche Reizmuster, insbesondere die "Coordinated Reset"-Stimulation (CR-Stimulation) appliziert, um eine dauerhafte Linderung zu erzielen. Die nicht-invasive CR-Stimulation kann mittels verschiedener Stimulationsmodi realisiert werden:

(i) durch sensorische Reizung, d. h. durch physiologische Reizung von Rezeptoren, wie z. B. akustische Reizung des Innenohrs, visuelle Reizung der Retina oder mechanische (z. B. vibrotaktile) oder thermische Reizung von Haut-, Unterhaut-, Muskel- und Sehnenrezeptoren;

(ii) durch Reizung peripherer Nerven (und zugehöriger Rezeptoren), z. B. mittels elektrischem Strom (z. B. transkutane Elektrostimulation), mittels Magnetfeldern (transdermale Magnetstimulation) oder mittels Ultraschall; und

(iii) durch Reizung des Gehirns oder Rückenmarks, z. B. mittels elektrischem Strom (z. B. externe kraniale bzw. transkranielle Neurostimulation), mittels Magnetfeldern (z. B. transkranielle Magnetstimulation) oder mittels Ultraschall.

Zur Behandlung des chronisch subjektiven tonalen bzw. engbandigen Tinnitus wird die akustische CR-Stimulation verwandt. Hierzu werden Therapietöne an den dominanten Tinnituston angepasst und im Sinne der CR-Stimulation appliziert, um eine lang anhaltende, das Ausschalten der Stimulation deutlich überdauernde Desynchronisation der krankhaft synchronen Aktivität bzw. sogar eine anhaltende Desynchronisation derselben zu erzielen. Die akustische CR-Stimulation zur Behandlung des Tinnitus bewirkt eine signifikante und deutlich ausgeprägte Abnahme der Symptomatik (vgl. P. A. Tass, I. Adamchic, H.-J. Freund, T. von Stackelberg, C. Hauptmann: Counteracting tinnitus by acoustic coordinated reset neuromodulation. Restorative Neurology and Neuroscience 30, 137-159 (2012)), eine signifikante Abnahme der krankhaften neuronalen Synchronisation in einem Netzwerk von auditorischen und nicht-auditorischen Hirnarealen (vgl. P. A. Tass, I. Adamchic, H.-J. Freund, T. von Stackelberg, C. Hauptmann: Counteracting tinnitus by acoustic coordinated reset neuromodulation. Restorative Neurology and Neuroscience 30, 137-159 (2012); I. Adamchic, T. Toth, C. Hauptmann, P. A. Tass: Reversing pathologically increased EEG power by acoustic CR neuromodulation. Human Brain Mapping 35, 2099-2118 (2014)), eine signifikante Abnahme der krankhaften Interaktionen zwischen unterschiedlichen Hirnarealen im selben (vgl. A. N. Silchenko, I. Adamchic, C. Hauptmann, P. A. Tass: Impact of acoustic coordinated reset neuromodulation on effective connectivity in a neural network of phantom sound. Neuroimage 77, 133-147 (2013)) sowie in unterschiedlichen (vgl. I. Adamchic, B. Langguth, C. Hauptmann, P. A. Tass: Abnormal brain activity and cross-frequency coupling in the tinnitus network. Frontiers in Neuroscience 8, 284 (2014)) Frequenzbereichen.

In analoger Weise lässt sich die Parkinsonsche Erkrankung mittels vibrotaktiler CR-Stimulation behandeln. Weitere Indikationen stellen z. B. Epilepsien, Funktionsstörungen nach Schlaganfall, chronische Schmerzsyndrome (mittels vibrotaktiler und/oder thermischer CR-Stimulation), Migräne (z. B. mittels visueller CR-Stimulation) dar. Des Weiteren lassen sich diese Erkrankungen mit transkranieller Magnetstimulation oder direkter elektrischer Stimulation des Gehirns oder direkter Hirnstimulation mittels Ultraschall behandeln.

Bei allen drei oben genannten Stimulationsmodalitäten (i) bis (iii) sollte zur Vermeidung von Nachteilen (aus jeweils unterschiedlichen, im Folgenden ausgeführten Gründen) bei geringen Reizstärken stimuliert werden. Dies sollte aber weder zu einer Verminderung der Wirksamkeit der Stimulation noch zu einer Verlängerung der Stimulationsdauer führen.

(i) Sensorische Reizung: Bei der sensorischen Stimulation ist es wichtig, die gewünschten Stimulationseffekte, z.

B. eine Phasenrücksetzung der krankhaft synchronisierten oszillatorischen Aktivität im Gehirn bzw. Rückenmark, bei möglichst geringer Reizstärke überhaupt erzielen zu können. Z. B. müssen bei der akustischen CR-Stimulation zur Behandlung des Tinnitus typischerweise schwerhörige Patienten behandelt werden. Die Stimulation mit lauten Tönen kann das Innenohr schädigen, kann die Kommunikation mit anderen erschweren sowie Warnlaute, wie z. B. eine Fahrzeughupe oder Fahrradklingel, verdecken bzw. vom Patienten infolge der vergleichsweise nahe an der Hörschwelle verlaufenden Unerträglichkeitsschwelle als deutlich unangenehm empfunden werden. Besonders problematisch kann die akustische Stimulation von Tinnituspatienten mit Hyperakusis sein, da derartige Patienten akustische Stimulation zum Teil als unangenehm oder sogar unerträglich empfinden. Zudem kann bei schwerhörigen Tinnituspatienten die laute Stimulation auch von der Umgebung des Patienten gehört und als störend empfunden werden. Bei der visuellen CR-Stimulation kann es insbesondere bei Migränepatienten zu unangenehmen Blendeffekten kommen. Bei der mechanischen, z. B. vibrotaktilen oder thermischen CR-Stimulation von Patienten mit chronischen Schmerzsyndromen, z. B. mit Morbus Sudeck oder Neuralgien, können schon leichte Berührungen oder Wärmereize als unangenehm oder sogar schmerzhaft empfunden werden. Wenn in derartigen Fällen z. B. über die kontralaterale Extremität oder Gesichts- bzw. Körperhälfte behandelt werden muss, ist die Reizwirkung infolge der Applikation in der gesunden Körperhälfte nicht stark ausgeprägt. Insgesamt ist es bei der sensorischen CR-Stimulation sehr vorteilhaft, wenn mit sehr geringen Reizstärken stimuliert werden kann, da sensorische Reize, wie Töne, Helligkeitsschwankungen von Transmissionsbrillen etc., die physiologische Reizverarbeitung stören können.

(ii) Elektrische oder magnetische Reizung peripherer Nerven: Um möglichst fokal stimulieren zu können und Nebenwirkungen, die durch die Mitreizung benachbarter Strukturen hervorgerufen werden, z. B. Muskelkontraktionen, Schmerzempfindungen etc., vermeiden zu können, ist es wichtig, möglichst geringe Reizstärken zu verwenden.

(iii) Elektrische oder magnetische Reizung des Gehirns oder Rückenmarks: Beide Stimulationsformen sind nicht sehr fokal. Z. B. die direkte elektrische Stimulation des Gehirns führt selbst im günstigsten Falle der Stimulation über eine Vielzahl von kleinen Elektroden und bei Verwendung aufwändiger Kopfmodelle neben einer fokalen starken Reizung zu einer Mitreizung von weit ausgedehnten Hirngebieten, die gerade bei chronischer Reizung unbedingt vermieden bzw. verringert werden sollte. In gleicher Weise sollte die Ultraschallreizung auf die tatsächlichen Zielgebiete im Gehirn beschränkt werden.

In all diesen Fällen ist es also nötig, bei möglichst geringen Reizstärken behandeln zu können, um die unerwünschte Mitreizung von Nicht-Zielarealen zu verringern. Dies allerdings führt häufig dazu, dass die Behandlung nicht hinreichend wirksam ist.

DE 10 2010 016404 A1 offenbart eine Vorrichtung mit einer nicht-invasiven ersten Stimulationseinheit zur Erzeugung von ersten Reizen, die bei einer Verabreichung an einen Patienten eine krankhaft synchrone Aktivität von Neuronen im Gehirn und/oder Rückenmark des Patienten unterdrücken, einer nicht-invasiven zweiten Stimulationseinheit zur Erzeugung von optischen und/oder akustischen und/oder taktilen und/oder vibratorischen und/oder thermischen zweiten Reizen, und einer Steuereinheit zur Steuerung der ersten und zweiten Stimulationseinheit, wobei die Erzeugung der ersten und zweiten Reize wahlweise in einem ersten oder einem zweiten Betriebsmodus erfolgt, und die Steuereinheit die erste und zweite Stimulationseinheit derart ansteuert, dass im ersten Betriebsmodus die Erzeugung von mindestens 60% der zweiten Reize zeitlich an die Erzeugung der ersten Reize gekoppelt ist und im zweiten Betriebsmodus die Erzeugung von mindestens 60% der zweiten Reize ohne die Erzeugung der ersten Reize erfolgt.

Der Erfindung liegt die Aufgabe zugrunde, eine Vorrichtung sowie ein Verfahren zur Stimulation von Neuronen anzugeben, mit denen sich trotz verringerter Reizstärke und ohne signifikant erhöhte Stimulationsdauer robuste therapeutische Effekte erzielen lassen.

Die der Erfindung zugrunde liegende Aufgabenstellung wird durch die Merkmale der unabhängigen Ansprüche gelöst. Vorteilhafte Weiterbildungen und Ausgestaltungen der Erfindung sind in den Unteransprüchen angegeben.

Die Erfindung wird nachfolgend in beispielhafter Weise unter Bezugnahme auf die Zeichnungen näher erläutert. In diesen zeigen:

Fig. 1 eine schematische Darstellung einer Vorrichtung zur Unterdrückung einer krankhaft synchronen und oszillatorischen neuronalen Aktivität und insbesondere zur Desynchronisierung von Neuronen mit einer krankhaft synchronen und oszillatorischen Aktivität gemäß einer ersten Ausgestaltung;

Fig. 2 eine schematische Darstellung eines fraktionierten Übergangs von einem ersten Stimulationsmodus in einen zweiten Stimulationsmodus;

Fig. 3A eine schematische Darstellung einer CR-Stimulation mit rasch variierenden Reizsequenzen;

Fig. 3B eine schematische Darstellung einer CR-Stimulation mit langsam variierenden Reizsequenzen;

Fig. 4 eine schematische Darstellung einer Vorrichtung zur Unterdrückung einer krankhaft synchronen und oszillatorischen neuronalen Aktivität und insbesondere zur Desynchronisierung von Neuronen mit einer krankhaft synchronen und oszillatorischen Aktivität gemäß einer zweiten Ausgestaltung;

Fig. 5 eine schematische Darstellung einer Vorrichtung zur akustischen Stimulation von Neuronen mit einer krankhaft synchronen und oszillatorischen neuronalen Aktivität;

Fig. 6 eine schematische Darstellung einer Vorrichtung zur visuellen Stimulation von Neuronen mit einer krankhaft synchronen und oszillatorischen neuronalen Aktivität;

Fig. 7 eine schematische Darstellung einer Vorrichtung zur taktilen, vibratorischen, thermischen, transkutanen elektrischen und/oder transkutanen magnetischen Stimulation und/oder Ultraschall-Stimulation von Neuronen mit einer krankhaft synchronen und oszillatorischen neuronalen Aktivität; und

Fig. 8 bis 11 Diagramme mit Simulationsergebnissen für verschiedene CR-Stimulationen.

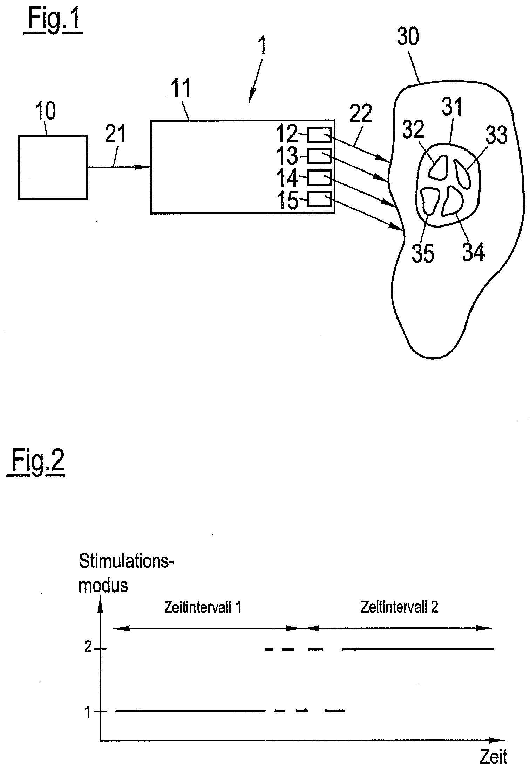

In Fig. 1 ist schematisch eine Vorrichtung 1 zur Stimulation von Neuronen mit einer krankhaft synchronen und oszillatorischen neuronalen Aktivität dargestellt. Die Vorrichtung 1 besteht aus einer Steuereinheit 10 und einer Stimulationseinheit 11, die über eine Mehrzahl von Stimulationskanälen Neuronen im Gehirn und/oder Rückenmark 30 eines Patienten stimuliert. Jeder Stimulationskanal ermöglicht die Stimulation eines anderen Zielgebiets im Gehirn und/oder Rückenmark 30 des Patienten, wobei die den Stimulationskanälen zugeordneten Zielgebiete nicht notwendigerweise disjunkt, d. h. vollständig voneinander getrennt sein müssen, sondern einander auch überlappen können. In Fig. 1 ist beispielhaft die Stimulation über vier Stimulationskanäle 12, 13, 14 und 15 dargestellt. Es kann selbstverständlich aber auch über eine andere Zahl von Stimulationskanälen, z. B. 2, 3, 5, 6 usw., stimuliert werden.

Während des Betriebs der Vorrichtung 1 führt die Steuereinheit 10 eine Steuerung der Stimulationseinheit 11 durch. Dazu erzeugt die Steuereinheit 10 Steuersignale 21, die von der Stimulationseinheit 11 entgegengenommen werden.

Die Stimulationseinheit 11 erzeugt anhand der Steuersignale 21 Reize 22 in den Stimulationskanälen 12 bis 15, die dem Patienten verabreicht werden. Die Reize 22 können sensorische Reize, z. B. akustische, visuelle, taktile, vibratorische, thermische, olfaktorische, gustatorische, transkutane elektrische, transkutane magnetische, transkranielle elektrische und/oder transkranielle magnetische Reize und/oder Ultraschall-Reize, sein. Insbesondere taktile und vibratorische Reize 22 werden auch gemeinsam appliziert und werden dann als vibrotaktile Reize 22 bezeichnet. Die Reize 22 können vom Patienten insbesondere bewusst wahrnehmbar sein. Die Reize 22 sind dazu ausgelegt, bei einer Verabreichung an den Patienten über die Stimulationskanäle 12 bis 15 die krankhaft synchrone und oszillatorische neuronale Aktivität zu unterdrücken und insbesondere die Neuronen mit der krankhaft synchronen und oszillatorischen Aktivität zu desynchronisieren.

Die Stimulationseinheit 11 und insbesondere auch die Steuer- und Analyseeinheit 10 sind nicht-invasive Einheiten, d. h., während des Betriebs der Vorrichtung 1 befinden sie sich außerhalb des Körpers des Patienten und werden nicht operativ in den Körper des Patienten implantiert.

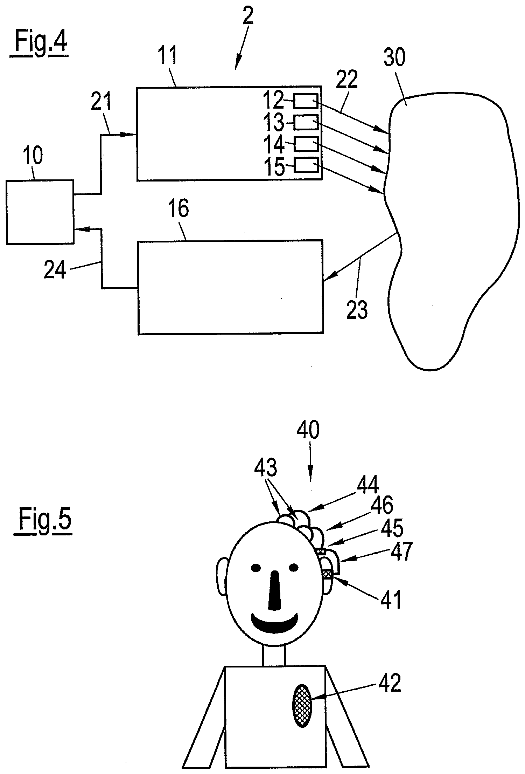

Die Vorrichtung 1 und die weiter unten, im Zusammenhang mit Fig. 4 beschriebene Vorrichtung 2 können insbesondere zur Behandlung von neurologischen oder psychiatrischen Erkrankungen eingesetzt werden, z. B. Morbus Parkinson, essentiellem Tremor, Tremor infolge von Multipler Sklerose sowie anderen pathologischen Tremores, Dystonie, Epilepsie, Depression, Bewegungsstörungen, Kleinhirnerkrankungen, Zwangserkrankungen, Demenzerkrankungen, Morbus Alzheimer, Tourette-Syndrom, Autismus, Funktionsstörungen nach Schlaganfall, Spastik, Tinnitus, Schlafstörungen, Schizophrenie, Reizdarm-Syndrom, Suchterkrankungen, Borderline-Persönlichkeitsstörung, Aufmerksamkeits-Defizit-Syndrom, Aufmerksamkeits-Defizit-Hyperaktivitäts-Syndrom, Spielsucht, Neurosen, Fresssucht, Magersucht, Essstörungen, Burnout-Syndrom, Fibromyalgie, Migräne, Cluster-Kopfschmerz, allgemeiner Kopfschmerz, Neuralgie, Ataxie, Tic-Störung oder Hypertonie, sowie weiteren Erkrankungen, die durch krankhaft gesteigerte neuronale Synchronisation gekennzeichnet sind.

Die vorstehend genannten Krankheiten können durch eine Störung der bioelektrischen Kommunikation von Neuronenverbänden, die in spezifischen Schaltkreisen zusammengeschlossen sind, verursacht werden. Hierbei generiert eine Neuronenpopulation anhaltend krankhafte neuronale Aktivität und möglicherweise eine damit verbundene krankhafte Konnektivität (Netzwerkstruktur). Dabei bildet eine große Anzahl von Neuronen synchron Aktionspotentiale aus, d. h., die beteiligten Neuronen feuern übermäßig synchron. Hinzu kommt, dass die kranke Neuronenpopulation eine oszillatorische neuronale Aktivität aufweist, d. h., die Neuronen feuern rhythmisch. Im Fall von neurologischen oder psychiatrischen Erkrankungen liegt die mittlere Frequenz der krankhaften rhythmischen Aktivität der betroffenen Neuronenverbände etwa im Bereich von 1 bis 30 Hz, kann aber auch außerhalb dieses Bereichs liegen. Bei gesunden Menschen feuern die Neuronen hingegen qualitativ anders, z. B. auf unkorrelierte Weise.

In Fig. 1 ist die Vorrichtung 1 während einer CR-Stimulation dargestellt. Im Gehirn und/oder Rückenmark 30 des Patienten weist mindestens eine Neuronenpopulation 31 eine wie vorstehend beschrieben krankhaft synchrone und oszillatorische neuronale Aktivität auf. Die Stimulationseinheit 11 erzeugt sensorische Reize 22, die von dem Patienten aufgenommen werden und über das Nervensystem an die krankhaft aktive Neuronenpopulation 31 im Gehirn und/oder Rückenmark 30 weitergeleitet werden. Die Reize 22 sind so ausgestaltet, dass die zeitversetzte (oder phasenverschobene) Stimulation über mindestens zwei Stimulationskanäle eine Desynchronisation der krankhaft synchronen Aktivität der Neuronenpopulation 31 bewirkt. Eine durch die Stimulation bewirkte Senkung der Koinzidenzrate der Neuronen kann zu einer Senkung der synaptischen Gewichte und somit zu einem Verlernen der Tendenz zur Produktion krankhaft synchroner Aktivität führen.

Die bei der CR-Stimulation verabreichten Reize 22 bewirken in der Neuronenpopulation 30 ein Zurücksetzen, einen sogenannten Reset, der Phase der neuronalen Aktivität der stimulierten Neuronen. Durch das Zurücksetzen wird die Phase der stimulierten Neuronen unabhängig von dem aktuellen Phasenwert auf einen oder nahe zu einem bestimmten Phasenwert, z. B. 0°, gesetzt (in der Praxis ist es nicht möglich, einen bestimmten Phasenwert exakt einzustellen, dies ist für eine erfolgreiche CR-Stimulation aber auch nicht erforderlich). Somit wird die Phase der neuronalen Aktivität der krankhaften Neuronenpopulation 31 mittels einer gezielten Stimulation kontrolliert. Da die krankhafte Neuronenpopulation 31 über die Stimulationskanäle 12 bis 15 an unterschiedlichen Stellen stimuliert wird, können die Phasen der neuronalen Aktivität der in Fig. 1 dargestellten Subpopulationen 32 bis 35 der krankhaften Neuronenpopulation 31 zu unterschiedlichen Zeitpunkten zurückgesetzt werden, indem die Reize 22 über die Stimulationskanäle 12 bis 15 zeitversetzt (oder phasenverschoben) appliziert werden. Im Ergebnis wird dadurch die krankhafte Neuronenpopulation 31, deren Neuronen zuvor synchron und mit gleicher Frequenz und Phase aktiv waren, in mehrere Subpopulationen mit unterschiedlichen Phasen aufgespalten. Beispielsweise wird über den Stimulationskanal 12 die Subpopulation 32 stimuliert, über den Stimulationskanal 13 wird die Subpopulation 33 stimuliert, über den Stimulationskanal 14 wird die Subpopulation 34 stimuliert und über den Stimulationskanal 15 wird die Subpopulation 35 stimuliert. Innerhalb jeder der Subpopulationen 32 bis 35 sind die Neuronen nach dem Zurücksetzen der Phase weiterhin synchron und feuern auch weiterhin mit derselben pathologischen Frequenz, aber jede der Subpopulationen 32 bis 35 weist bezüglich ihrer neuronalen Aktivität die Phase auf, die ihr durch den im jeweiligen Stimulationskanal 12 bis 15 generierten Reiz 22 aufgezwungen wurde. Dies bedeutet, dass die neuronalen Aktivitäten der einzelnen Subpopulationen 32 bis 35 nach dem Zurücksetzen ihrer Phasen weiterhin einen in etwa sinusförmigen Verlauf mit derselben pathologischen Frequenz haben, aber unterschiedliche Phasen.

Bedingt durch die krankhafte Interaktion zwischen den Neuronen ist der durch die Stimulation erzeugte Zustand mit mindestens zwei Subpopulationen instabil, und die gesamte Neuronenpopulation 31 nähert sich schnell einem Zustand kompletter Desynchronisation, in welchem die Neuronen unkorreliert feuern. Der gewünschte Zustand, d. h. die komplette Desynchronisation, ist somit nach der zeitversetzten (oder phasenverschobenen) Applikation der phasenrücksetzenden Reize 22 nicht sofort vorhanden, sondern stellt sich meist innerhalb weniger Perioden oder gar in weniger als einer Periode der pathologischen Frequenz ein.

Eine Theorie zur Erklärung des Stimulationserfolgs basiert darauf, dass die letztlich gewünschte Desynchronisation durch die krankhaft gesteigerte Interaktion zwischen den Neuronen erst ermöglicht wird. Hierbei wird ein Selbstorganisationsprozess ausgenutzt, der für die krankhafte Synchronisation verantwortlich ist. Derselbe bewirkt, dass auf eine Aufteilung einer Gesamtpopulation 31 in Subpopulationen 32 bis 35 mit unterschiedlichen Phasen eine Desynchronisation folgt. Im Gegensatz dazu würde ohne krankhaft gesteigerte Interaktion der Neuronen keine Desynchronisation erfolgen.

Darüber hinaus kann durch die CR-Stimulation eine Neuorganisation der Konnektivität der gestörten neuronalen Netzwerke erzielt werden, so dass lang anhaltende therapeutische Effekte bewirkt werden können. Der erzielte synaptische Umbau ist von großer Bedeutung für die wirksame Behandlung neurologischer oder psychiatrischer Erkrankungen.

Bei der CR-Stimulation werden üblicherweise die Reize 22 an verschiedenen Stellen des Gehirns und/oder Rückenmarks 30 zu unterschiedlichen Zeiten im Rahmen einer sogenannten Sequenz appliziert. In dem vorstehenden Beispiel wird innerhalb einer Sequenz in jedem der Stimulationskanäle 12 bis 15 genau ein Reiz 22 erzeugt, so dass an jedem der unterschiedlichen Stimulationsorte genau einmal gereizt wird, d. h., jede der Subpopulationen 32 bis 35 wird innerhalb einer Sequenz genau einmal gereizt. Über einen weiten Bereich der Reizstärke ist die CR-Stimulation mit langsam variierender Sequenz der CR-Stimulation mit fester Sequenz oder mit rasch, z. B. von Stimulationszyklus zu Stimulationszyklus variierender Sequenz überlegen. Im Bereich sehr geringer Reizstärken führt die CR-Stimulation zu keinen anhaltenden therapeutischen Effekten, unabhängig davon, ob und wie die Sequenz variiert wird.

Wenn die CR-Stimulation mit rasch variierender Sequenz bei sehr niedriger Reizstärke appliziert wird, kommt es unter Stimulation zu einem desynchronisierenden Effekt, der jedoch typischerweise nach dem Ende der Stimulation nicht lange anhält. Bei einer CR-Stimulation mit fester oder mit langsam variierender Sequenz kommt es bei sehr niedriger Reizstärke typischerweise zu keinerlei desynchronisierendem Effekt, weder während noch nach der Stimulation.

Der Erfindung liegt folgende überraschende Beobachtung zugrunde: Eine Epoche mit einer desynchronisierenden Stimulation mit einer rasch variierenden Sequenz hat bei einer niedrigen Reizstärke zwar für sich genommen keinen lang anhaltenden desynchronisierenden Effekt, aber in Kombination mit einer Epoche mit einer desynchronisierenden Stimulation mit langsam variierender Sequenz höherer Reizstärke bewirkt sie einen lang anhaltenden desynchronisierenden Effekt, der zumindest gleichwertig oder aber sogar signifikant besser ist als der Effekt, welcher durch zwei aufeinander folgende Epochen mit einer desynchronisierenden Stimulation (mit fester, rasch variierender oder langsam variierender Sequenz) mit der höheren Reizstärke erzielt wird.

Die Erfindung nutzt folglich eine Zwei-Stufen-Stimulation, bei der in der ersten Stufe mit rasch variierender Sequenz bei einer geringen Reizstärke stimuliert wird und in der zweiten Stufe mit langsam variierender Sequenz bei einer höheren Reizstärke stimuliert wird. Zur Realisierung der beiden Stimulationsstufen kann die Stimulationseinheit 11 in zwei verschiedenen Stimulationsmodi (oder Betriebsmodi) betrieben werden. Während zumindest 75% der Zeit eines ersten Zeitintervalls betreibt die Steuereinheit 10 die Stimulationseinheit 11 in einem ersten Stimulationsmodus. Im ersten Stimulationsmodus steuert die Steuereinheit 10 die Stimulationseinheit 11 derart an, dass die Stimulationseinheit 11 Sequenzen von Reizen 22 repetitiv erzeugt und die Reihenfolge der Stimulationskanäle 12 bis 15, in denen die Reize 22 innerhalb einer Sequenz erzeugt werden, für höchstens 5 nacheinander generierte Sequenzen konstant ist und danach variiert wird, wobei die Stärke der Reize 22 im ersten Stimulationsmodus kleiner oder gleich einer vorgegebenen Reizstärke ist. Auf das erste Zeitintervall folgt ein zweites Zeitintervall. Insbesondere kann sich das zweite Zeitintervall unmittelbar dem ersten Zeitintervall, d. h. ohne eine dazwischen liegende Pause anschließen. Während mindestens 75% der Zeit des zweiten Zeitintervalls betreibt die Steuereinheit 10 die Stimulationseinheit in dem zweiten Stimulationsmodus. Im zweiten Stimulationsmodus steuert die Steuereinheit 10 die Stimulationseinheit 11 derart an, dass die Stimulationseinheit 11 Sequenzen von Reizen 22 repetitiv erzeugt und die Reihenfolge der Stimulationskanäle 12 bis 15, in denen die Reize 22 innerhalb einer Sequenz erzeugt werden, für mindestens 25 nacheinander generierte Sequenzen konstant ist und danach variiert wird. Die Stärke der Reize 22 im zweiten Stimulationsmodus beträgt mindestens das 1,3-fache der vorgegebenen Reizstärke.

Die vorstehend beschriebene Stimulationsform ist hinreichend robust, so dass es für den gewünschten Stimulationserfolg ausreichend ist, wenn die Stimulationseinheit 11 für nur 75% der Zeit des ersten Zeitintervalls in dem ersten Stimulationsmodus und für nur 75% der Zeit des zweiten Zeitintervalls in dem zweiten Stimulationsmodus betrieben wird. Selbstverständlich können die Betriebszeiten des ersten und des zweiten Stimulationsmodus in dem jeweiligen Zeitintervall auch erhöht werden. Beispielsweise kann die Stimulationseinheit 11 während des vollständigen ersten Zeitintervalls im ersten Stimulationsmodus und während des vollständigen zweiten Zeitintervalls im zweiten Stimulationsmodus betrieben werden. Während Zeitspannen, in denen die Stimulationseinheit 11 während des ersten Zeitintervalls nicht im ersten Stimulationsmodus oder während des zweiten Zeitintervalls nicht im zweien Stimulationsmodus betrieben wird, kann entweder keine Stimulation oder eine Stimulation, die insbesondere anders ausgeführt sein kann als die in dieser Anmeldung beschriebene Stimulation, erfolgen.

Es kann für den Patienten vorteilhaft sein, den Wechsel vom ersten Stimulationsmodus in den zweiten Stimulationsmodus nicht abrupt, sondern fraktioniert durchzuführen, wie es beispielhaft in Fig. 2 dargestellt ist. Ein abrupter Wechsel von einer unterschwelligen Stimulationsstärke im ersten Stimulationsmodus zu einer überschwelligen Stimulationsstärke im zweiten Stimulationsmodus kann sehr unangenehm, z. B. schmerzhaft sein. Um diesen Übergang angenehmer zu gestalten, kann man Gewöhnungseffekte ausnutzen, indem man im Rahmen des Übergangs vom ersten Zeitintervall zum zweiten Zeitintervall mehrmals zwischen den beiden Stimulationsmodi hin- und herschaltet. Das Ausmaß der Nebenwirkungen, z. B. Schmerzen, hängt nicht nur von der Stimulationsstärke, sondern auch von der Dauer der Reizapplikation ab. Durch Applikation kurzer Epochen im zweiten Stimulationsmodus kann das Einsetzen der Nebenwirkungen deutlich abgeschwächt werden. Es kann sogar zu Gewöhnungseffekten kommen, so dass die Nebenwirkungen im später dauerhaft applizierten zweiten Stimulationsmodus geringer ausfallen als ohne den fraktionierten Übergang. Die Dauer zwischen dem Hin- und Herschalten zwischen dem ersten und dem zweiten Stimulationsmodus kann im Rahmen des Übergangs zeitlich variieren, z. B. zunehmen, wie dies beispielhaft in Fig. 2 gezeigt ist.

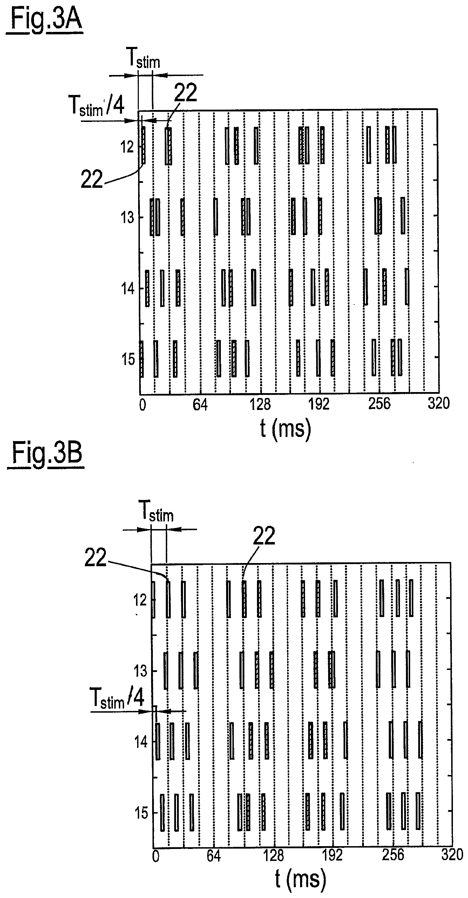

Ausführungsbeispiele für Stimulationen im ersten und zweiten Stimulationsmodus sind in Fig. 3A und 3B gezeigt.

Fig. 3A zeigt eine CR-Stimulation, bei der in vier Stimulationskanälen 12 bis 15 repetitiv rasch variierende Sequenzen von Reizen 22 im ersten Stimulationsmodus erzeugt werden. In Fig. 3A sind untereinander die in den Stimulationskanälen 12 bis 15 erzeugten Reize 22 gegen die Zeit t aufgetragen. Die Sequenzen werden in einem vorgegebenen Zeitraster, das aus aufeinanderfolgenden Zyklen besteht, erzeugt. Die einzelnen Zyklen sind in Fig. 3A durch gestrichelte Linien voneinander abgegrenzt. Jeder Zyklus weist die Länge $T_{stim}$ auf. In jedem Zyklus, in dem eine Stimulation erfolgt, wird in den Stimulationskanälen 12 bis 15 zusammen genau eine Sequenz von Reizen 22 erzeugt und in jedem Stimulationskanal 12 bis 15 wird genau einen Reiz 22 erzeugt, d. h., jede Sequenz besteht in dem vorliegenden Beispiel aus einer Abfolge von vier zeitversetzten Reizen 22, die insbesondere in jeweils unterschiedlichen Stimulationskanälen 12 bis 15 generiert werden, wobei sich der Zeitversatz insbesondere auf die Anfangszeitpunkte der Reize 22 beziehen kann. Zu Beginn jedes Zyklus wird in dem vorliegenden Beispiel die Reihenfolge der Stimulationskanäle 12 bis 15, in denen die Reize 22 innerhalb der jeweiligen Sequenz erzeugt werden, variiert. Eine unterschiedliche Füllung der in Fig. 3A gezeigten Balken, welche die Reize 22 symbolisieren, zeigt eine Variation der Reihenfolge an. Beispielsweise werden die Reize 22 in den Stimulationskanälen 12 bis 15 in dem ersten in Fig. 3A gezeigten Zyklus in der Reihenfolge 15-12-14-13 erzeugt. Im zweiten Zyklus lautet die Reihenfolge 15-13-14-12 und im dritten Zyklus lautet die Reihenfolge 12-15-14-13.

Wie oben beschrieben ist vorgesehen, dass die Sequenzen für maximal 5 nacheinander generierte Sequenzen gleich bleiben und danach geändert werden. Weiterhin kann die Variation der Sequenzen mit einem konstanten Rhythmus erfolgen, d. h., eine Variation findet beispielsweise stets nach $i_{Modus\_1}$ Zyklen statt, wobei $i_{Modus\_1}$ eine ganze Zahl von 1 bis 5 ist. Alternativ kann die Anzahl der Zyklen, nach der die Sequenz variiert wird, gemäß stochastischen oder deterministischen oder gemischt stochastisch-deterministischen Regeln bestimmt werden.

Gemäß einer Ausgestaltung wird bei der in Fig. 3A gezeigten CR-Stimulation nur die Reihenfolge der Stimulationskanäle 12 bis 15, in denen die Reize 22 innerhalb der jeweiligen Sequenz erzeugt werden, variiert. Alle übrigen Stimulationsparameter können während der CR-Stimulation konstant bleiben.

Die Variation der Sequenzen kann z. B. stochastisch oder deterministisch oder gemischt stochastisch-deterministisch erfolgen.

Es kann vorgesehen sein, dass die CR-Stimulation im ersten Stimulationsmodus kontinuierlich erfolgt, d. h., in aufeinander folgenden Zyklen werden stets Sequenzen von Reizen 22 erzeugt. Alternativ können aber auch Pausen während der CR-Stimulation, insbesondere während ganzer Zyklen, eingehalten werden. So können während $n_{Modus\_1}$ aufeinanderfolgenden Zyklen Reize 22 erzeugt werden und während der darauffolgenden $m_{Modus\_1}$ Zyklen keine Reize 22 erzeugt werden, die dazu ausgelegt sind, die krankhaft synchrone und oszillatorische neuronale Aktivität zu desynchronisieren, wobei $n_{Modus\_1}$ und $m_{Modus\_1}$ nicht-negative ganze Zahlen sind. Das Muster aus $n_{Modus\_1}$ Zyklen mit Stimulation und $m_{Modus\_1}$ Zyklen ohne Stimulation kann periodisch fortgesetzt werden. Für die in Fig. 3A gezeigte beispielhafte Stimulationsform gilt $n_{Modus\_1} = 3$ und $m_{Modus\_1} = 2$.

Es ist denkbar, dass andere Reize, die nicht dazu ausgelegt sind, die krankhaft synchrone und oszillatorische neuronale Aktivität zu unterdrücken, während der Stimulationspausen insbesondere mit der Stimulationseinheit 11 appliziert werden. Alternativ erzeugt die Stimulationseinheit 11 während der Stimulationspausen keinerlei Reize.

Sofern vorgesehen ist, die Sequenzen nach einer vorgegebenen Anzahl $i_{Modus\_1}$ von Sequenzen zu variieren ($i_{Modus\_1} \leq 5$), werden gemäß einer Ausgestaltung die Zyklen ohne Stimulation nicht mitgezählt, d. h., es findet bei dieser Ausgestaltung eine Variation der Reihenfolge der Stimulationskanäle 12 bis 15, in denen die Reize 22 innerhalb der jeweiligen Sequenz erzeugt werden, erst dann statt, wenn tatsächlich in $i_{Modus\_1}$ Zyklen jeweils eine Sequenz von Reizen 22 appliziert wurde.

Jeder der vier Stimulationskanäle 12 bis 15 stimuliert eine jeweilige der in Fig. 1 dargestellten Subpopulationen 32 bis 34 der krankhaften Neuronenpopulation 31. Während der maximal 5 Zyklen, in denen die Sequenzen konstant sind, wird in jedem der Stimulationskanäle 12 bis 15 der Reiz 22 periodisch mit der Periode $T_{stim}$ appliziert. Die Reize 22 bewirken eine Phasenrücksetzung der neuronalen Aktivität der jeweils stimulierten Subpopulation. Ferner beträgt die zeitliche Verzögerung zwischen innerhalb einer Sequenz zeitlich direkt aufeinanderfolgenden, in unterschiedlichen Stimulationskanälen erzeugten Reizen 22 $T_{stim}/4$, da in dem vorliegenden Ausführungsbeispiel vier Stimulationskanäle 12 bis 15 für die CR-Stimulation eingesetzt werden. Für den allgemeinen Fall von P für die Stimulation verwendeten Stimulationskanälen würde die zeitliche Verzögerung zwischen innerhalb einer Sequenz zeitlich direkt aufeinanderfolgenden, in unterschiedlichen Stimulationskanälen erzeugten Reizen 22 $T_{stim}/P$ betragen (von diesem Wert kann auch um z. B. bis zu $\pm$ 5%, $\pm$ 10% oder $\pm$ 20% abgewichen werden). Die zeitliche Verzögerung $T_{stim}/P$ kann sich auf die Anfangszeitpunkte der Reize 22 beziehen. Die in unterschiedlichen Stimulationskanälen erzeugten Reize 22 können bis auf die unterschiedlichen Startzeitpunkte identisch sein.

Die Periode $T_{stim}$, die zum einen die Dauer eines Zyklus und zum anderen die Periode angibt, mit der gleich bleibende Sequenzen sowie die in einem jeweiligen Stimulationskanal 12 bis 15 generierten Reize 22 wiederholt werden, kann nahe bei der mittleren Periode der pathologischen Oszillation der Neuronenpopulation 31 mit der krankhaft synchronen und oszillatorischen neuronalen Aktivität liegen bzw. um bis zu $\pm$ 5%, $\pm$ 10% oder $\pm$ 20% von der mittleren Periode abweichen. Typischerweise liegt die Frequenz $f_{stim} = 1/T_{stim}$ im Bereich von 1 bis 30 Hz. Die Periode der pathologischen Oszillation der zu stimulierenden Neuronenpopulation 31 kann, beispielsweise mittels EEG, gemessen werden. Es ist aber auch möglich, für die Periode der pathologischen Oszillation Literatur- oder Erfahrungswerte, die sich auf die jeweilige, zu behandelnde Krankheit beziehen, zu verwenden.

Die phasenrücksetzenden Reize 22 können beispielsweise Einzelreize oder auch zusammengesetzte Reize sein. Beispielsweise kann jeder Reiz 22 aus einem Pulszug mit 2 bis 100, insbesondere 2 bis 10 Einzelpulsen bestehen. Innerhalb eines Pulszugs werden die Einzelpulse ohne Unterbrechung mit einer Frequenz im Bereich von 50 bis 500 Hz, insbesondere im Bereich von 100 bis 150 Hz, wiederholt. Die Pulse eines Pulszugs können identisch sein. Je nach Stimulationsart kann es sich um einen akustischen, visuellen, taktilen, vibratorischen (insbesondere vibrotaktilen), thermischen, olfaktorischen, gustatorischen, transkutanen elektrischen, transkutanen magnetischen, transkraniellen elektrischen und/oder transkraniellen magnetischen Pulszug und/oder einen Ultraschall-Pulszug handeln.

Die Stärke der Reize 22, d. h. die Amplitude der Reize 22, ist im ersten Stimulationsmodus kleiner oder gleich einer vorgegebenen Reizstärke. Die vorgegebene Reizstärke kann insbesondere unterschwellig in dem Sinne sein, dass die Reize 22 nur während der Stimulation desynchronisierende Effekte haben, die das Ende der Stimulation jedoch nicht überdauern, d. h., nach dem Ende der Stimulation mit den Reizen 22, deren Reizstärke die vorgegebene Reizstärke nicht übersteigt, verschwindet der desynchronisierende Effekt.

Grundsätzlich kann es möglich sein, mittels der Stimulationseinheit 11 über eine beliebige Anzahl L von Stimulationskanälen zu stimulieren (L ≥ 2), jedoch müssen bei einer Stimulation nicht zwingend allen L Stimulationskanälen Reize 22 erzeugt werden, es können beispielsweise auch in nur einer Auswahl von P der L Stimulationskanäle die Reize 22 erzeugt werden (2 ≤ P ≤ L). Bei P Stimulationskanälen ergeben sich P! mögliche unterschiedliche Sequenzen, wobei bei jeder dieser Sequenzen in jedem der P Stimulationskanäle genau ein Reiz 22 erzeugt wird. Es ist denkbar, alle P! möglichen Sequenzen für die Stimulation heranzuziehen oder aus der Menge der P! möglichen Sequenzen eine Untermenge für die Stimulation auszuwählen. Diese Untermenge kann auch in der Zeit gemäß stochastisch oder deterministisch oder gemischt stochastisch-deterministisch Regeln variieren. Die Abfolge der Sequenzen kann zufällig sein oder vor Beginn oder auch während der Stimulation festgelegt werden.

Durch die Stimulation im ersten Stimulationsmodus wird die Neuronenpopulation 31 in einen Zustand gebracht, in dem sie für die nachfolgende Stimulation im zweiten Stimulationsmodus mit langsam variierender Sequenz und höherer Reizstärke deutlich empfänglicher ist.

Die Stimulation im zweiten Stimulationsmodus kann bis auf die Anzahl der Zyklen, nach denen die Sequenz variiert wird, und die Reizstärke die gleichen Ausgestaltungen aufweisen, wie die oben im Zusammenhang mit Fig. 3A erläuterte Stimulation im ersten Stimulationsmodus. Im Folgenden werden die Unterschiede der Stimulation im zweiten Stimulationsmodus gegenüber der Stimulation im ersten Stimulationsmodus anhand von Fig. 3B erläutert.

Fig. 3B zeigt eine CR-Stimulation, bei der in den vier Stimulationskanälen 12 bis 15 repetitiv langsam variierende Sequenzen von Reizen 22 im zweiten Stimulationsmodus erzeugt werden. Die Reihenfolge der Stimulationskanäle 12 bis 15, in denen innerhalb einer Sequenz die Reize 22 erzeugt werden, wird für mindestens 25 nacheinander generierte Sequenzen konstant gehalten und erst danach variiert. Es ist weiterhin denkbar, die Wiederholung derselben Sequenz zu erhöhen und die Reihenfolge, in welcher in den Stimulationskanälen 12 bis 15 pro Zyklus die Reize 22 erzeugt werden, im zweiten Stimulationsmodus für beispielsweise mindestens 30 oder mindestens 35 nacheinander generierte Sequenzen konstant zu halten. Es sei an dieser Stelle noch darauf hingewiesen, dass in Fig. 3B aus Gründen der Veranschaulichung die Sequenzen bereits nach weniger als 25 Sequenzen variiert werden. Dies ist jedoch lediglich als eine vereinfachte Darstellung einer im Vergleich zu Fig. 3A langsamen Sequenzvariation zu verstehen.

Die Variation der Sequenzen kann im zweiten Stimulationsmodus mit einem konstanten Rhythmus erfolgen, d. h., eine Variation findet beispielsweise stets nach $i_{Modus\_2}$ Zyklen statt, wobei $i_{Modus\_2} \geq 25$ gilt. Alternativ kann die Anzahl der Zyklen, nach der die Sequenz variiert wird, gemäß stochastischen oder deterministischen oder gemischt stochastisch-deterministischen Regeln bestimmt werden.

Wie bei der Stimulation im ersten Stimulationsmodus kann auch bei der Stimulation im zweiten Stimulationsmodus nur die Reihenfolge der Stimulationskanäle, in denen pro Sequenz die Reize 22 erzeugt werden, variiert werden. Alle übrigen Stimulationsparameter können während der Stimulation konstant bleiben.

Die Variation der Sequenzen kann z. B. stochastisch oder deterministisch oder gemischt stochastisch-deterministisch erfolgen.

Die CR-Stimulation kann im zweiten Stimulationsmodus kontinuierlich erfolgen, d. h., in aufeinander folgenden Zyklen werden stets Sequenzen von Reizen 22 erzeugt. Alternativ können aber auch Pausen während der CR-Stimulation, insbesondere während ganzer Zyklen, eingehalten werden. So können während $n_{Modus\_2}$ aufeinanderfolgenden Zyklen Reize 22 erzeugt werden und während der darauffolgenden $m_{Modus\_2}$ Zyklen keine Reize 22 erzeugt werden, die dazu ausgelegt sind, die krankhaft synchrone und oszillatorische neuronale Aktivität zu desynchronisieren, wobei $n_{Modus\_2}$ und $m_{Modus\_2}$ nicht-negative ganze Zahlen sind. Das Muster aus $n_{Modus\_2}$ Zyklen mit Stimulation und $m_{Modus\_2}$ Zyklen ohne Stimulation kann periodisch fortgesetzt werden. Für die in Fig. 3B gezeigte beispielhafte Stimulationsform gilt $n_{Modus\_2} = 3$ und $m_{Modus\_2} = 2$. Die Werte für $n_{Modus\_2}$ und $m_{Modus\_2}$ des zweiten Stimulationsmodus können, müssen aber nicht identisch sein mit den Werten für $n_{Modus\_1}$ bzw. $m_{Modus\_1}$ des ersten Stimulationsmodus.

Es ist denkbar, dass andere Reize, die nicht dazu ausgelegt sind, die krankhaft synchrone und oszillatorische neuronale Aktivität zu unterdrücken, während der Stimulationspausen insbesondere mit der Stimulationseinheit 11 appliziert werden. Alternativ erzeugt die Stimulationseinheit 11 während der Stimulationspausen keinerlei Reize.

Sofern vorgesehen ist, die Sequenzen nach einer vorgegebenen Anzahl $i_{Modus\_2}$ von Sequenzen zu variieren ($i_{Modus\_2} \geq 25$), werden gemäß einer Ausgestaltung die Zyklen ohne Stimulation nicht mitgezählt, d. h., es findet bei dieser Ausgestaltung eine Variation der Reihenfolge der Stimulationskanäle 12 bis 15, in denen die Reize 22 generiert werden, erst dann statt, wenn tatsächlich in $i_{Modus\_2}$ Zyklen jeweils eine Sequenz von Reizen 22 appliziert wurde.

Die Stärke der Reize 22, d. h. die Amplitude der Reize 22, beträgt im zweiten Stimulationsmodus mindestens das 1,3-fache der vorgegebenen Reizstärke. Die Stärke der Reize 22 kann insbesondere so groß sein, dass man einen ausgeprägten und anhaltenden therapeutischen und/oder desynchronisierenden Effekt erzielen würde, wenn man die Reize 22 während der gesamten Stimulationsdauer, d. h. während der ersten und der zweiten Zeitspanne, applizieren würde. Gemäß einer Ausgestaltung ist die untere Grenze für die Reizstärke im zweiten Stimulationsmodus größer als das 1,3-fache der vorgegebenen Reizstärke und beträgt das 1,5- oder 1,7-fache der vorgegebenen Reizstärke.

Bei der hier beschriebenen Zwei-Stufen-Stimulation wird die Reizstärke ohne Verlust oder Einschränkung der Wirksamkeit aufdosiert. Während der ersten Stufe, d. h. im ersten Stimulationsmodus, genügt eine unterschwellige Reizstärke, wodurch ungewünschte Effekte deutlich reduziert werden können. Durch die Stimulation im ersten Stimulationsmodus wird die Neuronenpopulation 31 in einen Zustand gebracht, in dem sie für die nachfolgend in der zweiten Stufe durchgeführte Stimulation im zweiten Stimulationsmodus deutlich empfänglicher ist. Die Zwei-Stufen-Stimulation ermöglicht folglich eine verbesserte Stimulationswirkung bei gleichzeitig reduzierten Nebenwirkungen und anderen unerwünschten Effekten.

Das zugrundeliegende Wirkprinzip der Zwei-Stufen-Stimulation, nämlich die Verstärkung der desynchronisierenden Wirkung der Stimulation mit langsam variierender Sequenz durch vorgeschaltete Stimulation mit rasch variierender Sequenz, gilt nicht nur bei unterschwelliger Reizstärke der Stimulation mit rasch variierender Sequenz. Vielmehr ist die Wirkung der Zwei-Stufen-Stimulation bei überschwelliger erster Stufe zumindest tendenziell besser als alle sonstigen Varianten der CR-Stimulation gleicher Intensität und Dauer. Für den Fall der ersten Stufe mit überschwelliger Reizstärke entfällt aber der besondere Vorteil, dass durch die Verwendung der unterschwelligen Stimulation Nebenwirkungen und andere unerwünschte Effekte vermieden oder zumindest reduziert werden können.

Die in Fig. 1 dargestellte Vorrichtung 1 zur Stimulation von Neuronen mit einer krankhaft synchronen und oszillatorischen neuronalen Aktivität führt eine sogenannte "open loop"-Stimulation durch, d. h., eine Stimulation ohne Sensoren, die zur Rückmeldung und/oder Steuerung der Stimulation verwendet werden.

Fig. 4 zeigt schematisch eine Vorrichtung 2 zur Stimulation von Neuronen mit einer krankhaft synchronen und oszillatorischen neuronalen Aktivität, mit der sich eine "closed loop"-Stimulation durchführen lässt. Die Vorrichtung 2 ist eine Weiterbildung der in Fig. 1 dargestellten Vorrichtung 1 und enthält genauso wie die Vorrichtung 1 eine Steuereinheit 10 und eine nicht-invasive Stimulationseinheit 11, welche die gleichen Funktionen und Eigenschaften wie die oben beschriebenen Steuer- und Stimulationseinheiten 10, 11 der Vorrichtung 1 aufweisen.

Darüber hinaus umfasst die Vorrichtung 2 eine Messeinheit 16. Der durch die Reize 22 erzielte Stimulationseffekt wird mit Hilfe der Messeinheit 16 überwacht. Die Messeinheit 16 nimmt ein oder mehrere am Patienten gemessene Messsignale 23 auf, wandelt diese gegebenenfalls in elektrische Signale 24 um und führt sie der Steuereinheit 10 zu. Insbesondere kann mittels der Messeinheit 16 die neuronale Aktivität in dem stimulierten Zielgebiet oder einem mit dem Zielgebiet verbundenen Gebiet gemessen werden, wobei die neuronale Aktivität dieses Gebiets mit der neuronalen Aktivität des Zielgebiets hinreichend eng korreliert. Mittels der Messeinheit 16 kann auch eine nicht-neuronale, z. B. muskuläre Aktivität oder die Aktivierung des autonomen Nervensystems, gemessen werden, sofern diese mit der neuronalen Aktivität des Zielgebiets hinreichend eng korreliert sind.

Die Messeinheit 16 enthält ein oder mehrere Sensoren, die es insbesondere ermöglichen, eine Ab- bzw. Zunahme der Amplitude der pathologischen oszillatorischen Aktivität nachzuweisen.

Als Sensoren können nicht-invasive Sensoren eingesetzt werden, z. B. chronisch oder intermittent genutzte Elektroenzephalographie (EEG)- oder Elektromyographie (EMG)-Elektroden oder Magnetenzephalographie (MEG)-Sensoren. Die neuronale Aktivität kann auch durch Detektion charakteristischer Bewegungsmuster wie Tremor, Akinese oder epileptische Anfälle mit Hilfe eines Akzelerometers oder Gyroskops oder indirekt durch Messung der Aktivierung des autonomen Nervensystems mittels Messung des Hautleitwiderstands ermittelt werden. Es können auch Befindlichkeitswerte, die vom Patienten in portable Geräte, z. B. Smartphones, eingegeben werden, zur Kontrolle des Stimulationserfolgs verwandt werden. Derartige Befindlichkeitswerte können auch über Kurzfragebögen ermittelt werden.

Alternativ, aber weniger bevorzugt können die Sensoren in den Körper des Patienten implantiert sein. Als invasive Sensoren können beispielsweise epikortikale Elektroden, Tiefenhirnelektroden zur Messung von z. B. lokalen Feldpotentialen, sub- oder epidurale Hirnelektroden, subkutane EEG-Elektroden und sub- oder epidurale Rückenmarkselektroden dienen.

Die Steuereinheit 10 verarbeitet die Signale 24, z. B. können die Signale 24 verstärkt und/oder gefiltert werden, und analysiert die verarbeiteten Signale 24. Die Steuereinheit 10 überprüft anhand der in Reaktion auf die Applikation der Reize 22 aufgenommenen Messsignale 23 den Stimulationserfolg.

Sobald insbesondere anhand der von der Messeinheit 16 aufgenommenen Messsignale 23 eine ausgeprägte Desynchronisation bzw. akute klinische Befundbesserung bzw. eine ausgeprägte Besserung der Befindlichkeit des Patienten festgestellt worden ist, kann, insbesondere mit Hilfe der Steuereinheit 10, von dem ersten Stimulationsmodus in den zweiten Stimulationsmodus umgeschaltet werden. Insbesondere kann eine mit der Steuereinheit 10 gekoppelte Eingabeeinheit vorgesehen sein, die von dem Patienten und/oder dem behandelnden Arzt bedient werden kann, und mit der vom ersten Stimulationsmodus in den zweiten Stimulationsmodus umgeschaltet werden kann.

Der Stimulationserfolg kann insbesondere mittels eines Schwellwertvergleichs überprüft werden. Je nachdem welche Signale zur Ermittlung des Stimulationserfolgs herangezogen werden, ergeben sich unterschiedliche Schwellwertvergleiche. Wird z. B. die krankhafte neuronale Synchronisation über die Sensoren der Messeinheit 16, z. B. EEG-Elektroden oder Tiefelektroden (als LFP-Signal), gemessen, reicht erfahrungsgemäß die Absenkung der Synchronisation um einen vorgegebenen Wert, z. B. um mindestens 20%, im Vergleich zur Situation ohne Stimulation, um einen ausreichenden Stimulationserfolg festzustellen und vom ersten in den zweiten Stimulationsmodus zu wechseln. Es können aber größere Werte, z. B. 50% und mehr, gewählt werden, um länger im ersten Stimulationsmodus und somit mit geringerer Reizstärke zu stimulieren.

Die klinische Befundbesserung wird anhand typischer, dem Fachmann bekannter Änderungen von klinischen Scores bzw. Fragebögen bestimmt. Hierzu werden z. B. die aus der Literatur bekannten Werte Delta S für einen "minimal clinically relevant change" (minimale klinisch relevante Änderung) oder auch größere Werte, z. B. 2 x Delta S, verwandt.

Zusätzlich zu der oben beschriebenen Regelung, die das Umschalten von dem ersten in den zweiten Stimulationsmodus bestimmt, kann eine weitere Regelung vorgesehen sein, die auf einer langsameren Zeitskala agiert. Wenn sich ein Therapieerfolg über einen vordefinierten Zeitraum, z. B. 1 Stunde, eingestellt hat, wird die Stimulation abgeschaltet. Der Therapieerfolg wird hierbei wie oben beschrieben gemessen, wobei die Schwellenwerte für einen ausreichenden Therapieerfolg vom Anwender voreingestellt werden können, z. B. eine Absenkung der anfänglichen Synchronisation um 80%. Wenn diese Schwellenwerte für eine vordefinierte Dauer, z. B. 60 s, wieder überschritten werden und/oder der Patient eine nicht mehr hinreichend gebesserte Befindlichkeit meldet, wird die Zwei-Stufen-Stimulation wie oben beschrieben neu gestartet.

Mit Hilfe der Messeinheit 16 der Vorrichtung 2 können Werte für die Längen des ersten Zeitintervalls und des zweiten Zeitintervalls für einen jeweiligen Patienten abgeschätzt werden, die benötigt werden, um den gewünschten Stimulationserfolg zu erzielen. Anschließend können diese Informationen für eine Anwendung mit der Vorrichtung 1, die über keine Messeinheit verfügt, verwendet werden. Grundsätzlich können die Längen des ersten und des zweiten Zeitintervalls im Minuten- oder Stundenbereich liegen.

Weiterhin lässt sich mit Hilfe der Messeinheit 16 gemäß einer Ausgestaltung die vorgegebene Reizstärke bestimmen, aus der sich die obere bzw. untere Grenze für die Reizstärken im ersten und zweiten Stimulationsmodus ergibt. Auch diese Information kann anschließend bei einer Anwendung mit der Vorrichtung 1 genutzt werden. Zur Bestimmung der vorgegebenen Reizstärke wird die Stimulationseinheit 11 z. B. im ersten Stimulationsmodus betrieben und die Stärke der Reize 22 wird ausgehend von Null solange erhöht, bis sich ein Akuteffekt einstellt, d. h. eine Verringerung der Synchronisation der stimulierten Neuronenpopulation 31, die jedoch nach der Beendigung der Stimulation wieder verschwindet. Aus der so gewonnenen Reizstärke kann die vorgegebene Reizstärke abgeleitet werden, indem die vorgegebene Reizstärke beispielsweise aus einem Bereich ausgewählt wird, dessen untere Grenze die Reizstärke, bei der eine Verringerung der Synchronisation der stimulierten Neuronenpopulation einsetzt, darstellt und dessen obere Grenze z. B. das 1,1-fache der vorstehenden Reizstärke ist.

Die einzelnen Komponenten der Vorrichtungen 1 und 2, insbesondere die Steuereinheit 10, die Stimulationseinheit 11 und/oder die Messeinheit 16, können baulich voneinander getrennt sein. Die Vorrichtungen 1 und 2 können daher auch als Systeme aufgefasst werden. Zur Durchführung ihrer Aufgaben kann die Steuereinheit 10 einen Prozessor, z. B. einen Mikrocontroller, enthalten. Die hierin beschriebenen Stimulationsverfahren können als Software-Code in einem der Steuereinheit 10 zugeordneten Speicher abgelegt sein.

Fig. 5 zeigt schematisch eine Vorrichtung 40 zur nicht-invasiven akustischen Stimulation von Neuronen mit einer krankhaft synchronen und oszillatorischen neuronalen Aktivität gemäß einer Ausführungsform der Erfindung. Akustische Reize, insbesondere akustische CR-Reize, werden dem Patienten über Ohr- oder Kopfhörer 41 oder anders ausgestaltete Lautsprecher verabreicht, wobei ein Ohrhörer ein im Ohrkanal platzierter Lautsprecher ist. Die hierzu verwendeten Steuersignale werden von einer Steuereinheit 42 generiert. Nicht-invasiv fixierte EEG-Elektroden 43, die über ein Kabel 44 verbunden sind, dienen der "closed loop"-Stimulation. Die entsprechende Verrechnung wird in einem kleinen Bauteil 45, das vorzugsweise einen Messverstärker enthält und über Kabel 46, 47 mit den EEG-Elektroden 43 bzw. dem Ohr- oder Kopfhörer 41 verbunden ist, und/oder in der eigentlichen, die Batterie bzw. den Akku beherbergenden Steuereinheit 42 durchgeführt. Die Steuereinheit 42 und das Bauteil 45 sind in der in Fig. 5 dargestellten Ausführungsform telemetrisch miteinander verbunden; in diesem Fall enthält das Bauteil 45 (oder ein mit ihm über Kabel verbundenes Bauteil) ebenso eine Batterie bzw. einen Akku. Alternativ können die Steuereinheit 42 und das Bauteil 45 auch über Kabel miteinander verbunden sein, so dass das Bauteil 45 über die Stromversorgung von der Steuereinheit 42 gespeist wird.

Sofern es sich bei den hierin beschriebenen Reizen 22 um akustische Reize 22 handelt, ist die Reizstärke durch die Lautstärke der Reize 22 gegeben.

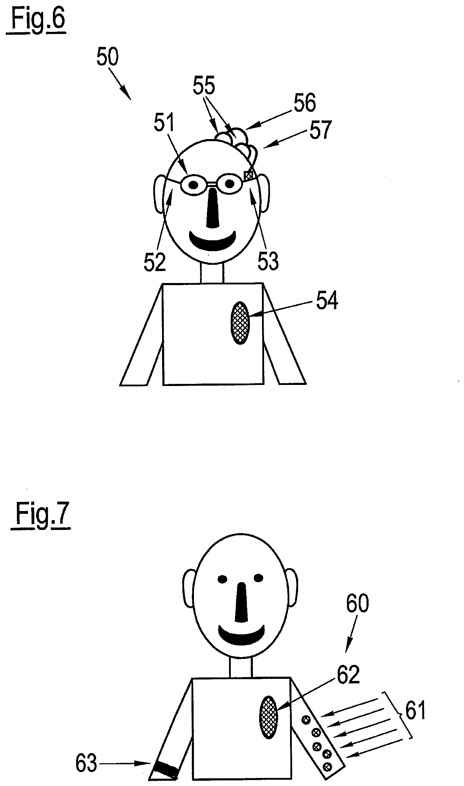

Fig. 6 zeigt schematisch eine Vorrichtung 50 zur nicht-invasiven visuellen Stimulation von Neuronen mit einer krankhaft synchronen und oszillatorischen neuronalen Aktivität gemäß einer Ausführungsform der Erfindung. Bei dieser Ausgestaltung trägt der Patient eine Stimulationsbrille 51, die z. B. über Bügel 52 am Kopf des Patienten befestigt ist. Ein Bauteil 53 enthält eine Verrechnungs- und Telemetrieeinheit. Letztere dient der Verbindung mit der eigentlichen, die Batterie bzw. den Akku beherbergenden Steuereinheit 54. Das Bauteil 53 und die Steuereinheit 54 sind telemetrisch miteinander verbunden; in diesem Fall enthält das Bauteil 53 (oder ein mit ihm über Kabel verbundenes Bauteil) ebenso eine Batterie bzw. einen Akku. Alternativ können das Bauteil 53 und die Steuereinheit 54 auch über Kabel miteinander verbunden sein. Nicht-invasiv fixierte EEG-Elektroden 55 dienen der "closed loop"-Stimulation. Die EEG-Elektroden 55 sind über Kabel 56, 57 mit dem Bauteil 53 verbunden.

Den von der Stimulationsbrille 51 erzeugten visuellen Reizen kann eine Leuchtstärken- bzw. Helligkeitsvariation (bzw. Variation der Lichtintensität oder Lichtstärke) zugrunde liegen, beispielsweise können sie als Pulse oder als Sequenzen von Pulsen mit variierter Leuchtstärke bzw. Helligkeit appliziert werden. Die visuellen Reize können je nach Ausgestaltung als Leuchtstärkenmodulation natürlicher visueller Reize, z. B. mittels einer homogenen oder segmentierten Transmissionsbrille, bei der spannungsabhängig die Transmission geregelt werden kann, als zusätzlich zu einem natürlichen visuellen Reiz auftretender, modulierter visueller Reiz, z. B. mittels einer partiell durchsichtigen Lichtbrille, oder als künstlicher visueller Helligkeitsreiz, z. B. mittels einer undurchsichtigen Lichtbrille, verabreicht werden. Die Stimulationsbrille 51 ist vorzugsweise in unterschiedliche Segmente unterteilt, deren Leuchtstärke bzw. Transmission bzw. Helligkeit getrennt gesteuert werden kann, um verschiedene Stellen der Retina unabhängig voneinander stimulieren zu können.

Fig. 7 zeigt schematisch eine Vorrichtung 60 zur nicht-invasiven taktilen, vibratorischen, thermischen, transkutanen elektrischen und/oder transkutanen magnetischen Stimulation und/oder Ultraschall-Stimulation von Neuronen mit einer krankhaft synchronen und oszillatorischen neuronalen Aktivität gemäß einer Ausführungsform der Erfindung. Die Vorrichtung 60 umfasst eine Stimulationseinheit 61, eine die Stimulationseinheit 61 ansteuernde Steuereinheit 62 und ein Akzelerometer (Beschleunigungsmesser) 63 zum Aufnehmen von Messsignalen. Die Stimulationseinheit 61 und das Akzelerometer 63 können mit der Steuereinheit 62 telemetrisch oder über Kabel verbunden sein.

Die Stimulationseinheit 61 umfasst eine Mehrzahl von Stimulationselementen zur Erzeugung von taktilen, vibratorischen, thermischen, transkutanen elektrischen und/oder transkutanen magnetischen Reizen und/oder Ultraschall-Reizen. Die Stimulationselemente sind derart ausgestaltet, dass sie auf die Haut des Patienten aufgesetzt werden können. Je nach Erkrankung bzw. betroffenen Körperpartien werden die Stimulationselemente in einer geeigneten Anordnung auf der Haut des Patienten befestigt, beispielsweise am Arm, am Bein, an der Hand und/oder am Fuß des Patienten. Die Mehrzahl von Stimulationselementen ermöglicht es, unterschiedliche rezeptive Bereiche der Haut über die einzelnen Stimulationselemente zeitlich und räumlich koordiniert zu stimulieren.

Stimulationselemente zur Erzeugung von taktilen und/oder vibratorischen Reizen sind beispielsweise Vibrationsaktoren, die mit einer Frequenz im Bereich von 1 bis 300 Hz und insbesondere 1 bis 60 Hz und vorzugsweise 100 bis 300 Hz in die Haut des Patienten eindrücken und dadurch die gewünschten Reize erzeugen. Im Fall von Vibrationsaktoren ist die Reizstärke durch die Amplitude der Vibrationsaktoren gegeben. Stimulationselemente zur Erzeugung thermischer Reize können beispielsweise Laser oder anders ausgestaltete Elemente zur Erzeugung von Wärme, insbesondere Wärmestrahlung, sein. Die Reizstärke ist hier durch die mit den thermischen Reizen erzeugte Temperatur gegeben.

Zur Erzeugung von transkutanen elektrischen Reizen werden typischerweise Elektroden auf der Haut des Patienten befestigt. Transkutane magnetische Reize können durch entsprechende Stimulationselemente zur Erzeugung magnetischer Reize, insbesondere stromdurchflossene Spulen, erzeugt werden. Ultraschall-Reize werden durch Stimulationselemente zur Erzeugung von Ultraschall-Wellen erzeugt. Bei den vorstehenden Stimulationsmodalitäten ist die Reizstärke durch die Stromstärke der transkutanen elektrischen Reize, die von den magnetischen Reizen am Stimulationsort erzeugte magnetische Feldstärke bzw. die Amplitude der Ultraschall-Wellen gegeben.

Bei der Applikation akustischer oder visueller Reize werden diese über mindestens ein Ohr bzw. mindestens ein Auge des Patienten aufgenommen. Die taktilen, vibratorischen, thermischen, transkutanen elektrischen und/oder transkutanen magnetischen Reize und/oder Ultraschall-Reize können von in oder unter der Haut gelegenen Rezeptoren aufgenommen und an das Nervensystem weitergeleitet werden. Zu diesen Rezeptoren zählen beispielsweise Merkel-Zellen, Ruffini-Körperchen, Meissner-Körperchen und Haarfollikelrezeptoren, die insbesondere als Rezeptoren für die taktilen Reize wirken. Die vibratorischen Reize zielen vorwiegend auf die Tiefensensibilität ab. Die vibratorischen Reize können von in der Haut, den Muskeln, dem Subkutangewebe und/oder den Sehnen des Patienten gelegenen Rezeptoren aufgenommen werden. Als Rezeptoren für die vibratorischen Reize seien beispielhaft die Vater-Pacini-Körperchen genannt, die Vibrationsempfindungen und Beschleunigungen vermitteln. Die thermischen Reize werden von den Thermorezeptoren der Haut aufgenommen. Dies sind Warmrezeptoren (auch Wärmerezeptoren, Warmsensoren oder Wärmesensoren genannt) und Kaltsensoren (auch Kältesensoren, Kaltrezeptoren oder Kälterezeptoren genannt).

In der Haut des Menschen liegen die Kaltsensoren mehr oberflächlich, die Warmrezeptoren etwas tiefer. Die transkutanen elektrischen und transkutanen magnetischen Reize sowie die Ultraschall-Reize wirken nicht spezifisch auf nur eine Gruppe von in oder unter der Haut gelegenen Rezeptoren und können darüber hinaus auch direkt Nervenfasern reizen.

Die gezielte Stimulation bestimmter Bereiche des Gehirns oder Rückenmarks wird durch die tonotope bzw. somatotope Zuordnung von Körperregionen zu diesen Bereichen ermöglicht. Beispielsweise werden akustische Reize im Innenohr in Nervenimpulse umgesetzt und über den Hörnerv zu dem auditorischen Cortex weitergeleitet. Durch die tonotope Anordnung des auditorischen Cortex wird bei der akustischen Stimulation des Innenohres mit einer bestimmten Frequenz ein bestimmter Teil des auditorischen Cortex aktiviert.

Bei der visuellen Stimulation werden unterschiedliche Stellen im Gesichtsfeld über die Linse des Auges auf unterschiedliche Stellen der Retina abgebildet. Die unterschiedlichen Stellen der Retina sind wiederum über den Sehnerv mit unterschiedlichen Neuronen im Gehirn verbunden. Folglich können mit den an unterschiedlichen räumlichen Orten applizierten Reizen jeweils unterschiedliche Neuronen stimuliert werden.

Aufgrund der somatotopen Gliederung der Nervenleitungsbahnen und zugehörigen Hirngebiete werden des Weiteren durch taktile, vibratorische, thermische, transkutane elektrische und/oder transkutane magnetische Reize und/oder Ultraschall-Reize, die an unterschiedlichen Stellen der Haut appliziert werden, unterschiedliche Neuronen stimuliert. Bei diesen Stimulationsformen können die Stimulationselemente beispielsweise am Fuß, Unterschenkel und Oberschenkel oder aber an der Hand, dem Unterarm und Oberarm des Patienten angebracht werden, um dadurch bestimmte Neuronen stimulieren zu können.

Bei der gustatorischen Reizung werden unterschiedliche Bereich der Zunge mit den entsprechenden Geschmacksqualitäten - süß, sauer, salzig, bitter und umami (japanisch für pikantes, würziges, bouillonartiges Aroma) - gereizt. Es ist aber auch möglich, die Zunge elektrisch zu reizen. In diesem Fall reizt man primär die Schleimhaut, welche im Homunculus (Repräsentation der Oberfläche des Menschen im sensomotorischen Cortex) eine erheblich große Repräsentation, d. h. ein erheblich großes zugeordnetes Areal, aktiviert. Aufgrund der somatotopen Gliederung der Nervenleitungsbahnen und zugehörigen Hirngebiete werden durch gustatorische Reize, die an unterschiedlichen Stellen der Zunge appliziert werden, unterschiedliche Neuronen stimuliert.

Ganz allgemein und nicht nur bezogen auf die hier beschriebenen Ausführungsbeispiele gilt Folgendes. Bei der akustischen Stimulation ist jeder Stimulationskanal einem jeweiligen unterschiedlichen Frequenzbereich zugeordnet, aus dem die Töne, die als akustische Reize in dem jeweiligen Stimulationskanal appliziert werden, ausgewählt werden. Bei der visuellen Stimulation werden die Stimulationskanäle durch unterschiedliche Stellen oder Bereiche im Gesichtsfeld des Patienten bestimmt. Die in einem jeweiligen Stimulationskanal erzeugten visuellen Reize werden an einer jeweiligen Stelle bzw. in einem jeweiligen Bereich des Gesichtsfelds erzeugt. Die Stimulationskanäle der taktilen, vibratorischen, thermischen, transkutanen elektrischen und/oder transkutanen magnetischen Reize und/oder Ultraschall-Reize werden durch die Stellen der Haut, welche mit den jeweiligen Stimulationselementen stimuliert werden, bestimmt. Folglich ist jeder Stimulationskanal einer jeweiligen Stelle bzw. einem jeweiligen Bereich der Haut zugeordnet.

Die Stimulationskanäle der gustatorischen Reize werden durch die Stellen der Zunge, welche mit den entsprechenden Geschmacksqualitäten oder elektrischen Reizen stimuliert werden, bestimmt. Bei einer olfaktorischen Stimulation verwendet man psychophysisch hinreichend disjunkte Geruchsreize, durch welche die Stimulationskanäle festgelegt würden. Die psychophysisch hinreichend disjunkten Geruchsreize könnten z. B. personalisiert, d. h. an den individuellen Patienten angepasst sein.

Bei der transkraniellen elektrischen und transkraniellen magnetischen Stimulation werden Elektroden bzw. Magnetfeldgeneratoren, insbesondere stromdurchflossene Spulen, am Körper, insbesondere am Kopf, des Patienten befestigt. Durch die Elektroden und Magnetfeldgeneratoren können Ströme bzw. Magnetfelder im Gehirn und/oder Rückenmark des Patienten erzeugt werden, wobei die Stärke dieser Ströme bzw. Magnetfelder die Reizstärke angibt. Je nach Anbringungsort der Elektroden bzw. Magnetfeldgeneratoren können unterschiedliche Zielgebiete im Gehirn und/oder Rückenmark stimuliert werden. Die Stimulationskanäle werden folglich durch die Stellen am Körper des Patienten, an denen die Elektroden bzw. Magnetfeldgeneratoren angebracht sind, bestimmt.

Die vorstehend beschriebene Stimulationseinheit kann demnach unterschiedliche Bereiche des Gehirns oder Rückenmarks über verschiedene Stimulationskanäle separat stimulieren, indem die applizierten Reize über Nervenleitungen an unterschiedliche Zielgebiete, die im Gehirn und/oder Rückenmark liegen, weitergeleitet werden. Die Zielgebiete können während der Stimulation mit eventuell unterschiedlichen und/oder zeitversetzten Reizen stimuliert werden.

Wie oben beschrieben bewirken die Reize 22 bei der CR-Stimulation ein Zurücksetzen, einen sogenannten Reset, der Phase der neuronalen Aktivität der stimulierten Neuronen. Mit Hilfe der von der Messeinheit 16 aufgenommenen Messsignale 23 kann die Phasenrücksetzung der einzelnen Reize 22 überprüft werden. Eine derartige Untersuchung kann vor der eigentlichen therapeutischen Zwei-Stufen-Neurostimulation vorgenommen werden.

Dazu wird über einen Sensor der Messeinheit 16 ein Signal gemessen, welches die Aktivität der über den j-ten Stimulationskanal stimulierten Subpopulation hinreichend repräsentiert. Man erhält dieses Signal entweder direkt von der Subpopulation über eine nicht-invasive Messung, z. B. über EEG- oder MEG-Elektroden, oder eine invasive Messung, z. B. über implantierte Elektroden, als Oberflächen-EEG oder als lokales Feldpotential über Tiefenelektroden. Das Signal kann auch indirekt über die Messung einer mit der Aktivität der stimulierten Subpopulation korrelierten Größe ermittelt werden. Hierzu eignen sich z. B. EEG-/MEG-/LFP-Signale der neuronalen Aktivität einer mit dieser Subpopulation eng gekoppelten anderen Neuronenpopulation oder zugehörige Elektromyographie-, Akzelerometer- oder Gyroskop-Signale.

Da neuronale Signale typischerweise rhythmische Aktivität in unterschiedlichen Frequenzbändern enthalten, ist es in solchen Fällen vorteilhaft, z. B. mittels Bandpassfilterung oder wavelet-Analyse oder empirical mode decomposition das Signal $x_j(t)$, welches die pathologische oszillatorische Aktivität der vom j-ten Stimulationskanal stimulierten Subpopulation repräsentiert, zu ermitteln.

Ein nur wenig aufwändiges Vorgehen, um eine Phasenrücksetzung zu überprüfen, besteht darin, die gemittelte Reizantwort zu bestimmen. Hierzu wird zu den Zeiten $\tau_1$, $\tau_2$, ..., $\tau_l$ ein Reiz mit identischen Reizparametern appliziert. Die Abstände zwischen den einzelnen Reizen $\tau_{k+1} - \tau_k$ sollten hinreichend groß und randomisiert, also nicht konstant sein, um Einschwingvorgänge zu vermeiden (vgl. P. A. Tass: Transmission of stimulus-locked responses in two coupled phase oscillators. Phys. Rev. E 69, 051909-1-24 (2004)). Typischerweise sollten die Abstände $\tau_{k+1} - \tau_k$ im Bereich von mindestens dem Zehnfachen, besser dem Hundertfachen der mittleren Periode des pathologischen Oszillation liegen. Die über alle I Test-Reize gemittelte Reizantwort wird gemäß folgender Gleichung berechnet:

$$\bar{x}_j(t) = \frac{1}{l}\sum_{k=1}^{l} x_j(\tau_k + t) \tag{1}$$

Sofern die Abstände $\tau_{k+1} - \tau_k$ zwischen den einzelnen Reizen hinreichend groß sind, erhält man im prä-Stimulus-Bereich, d. h. im Bereich vor der Applikation eines jeweiligen Reizes, keine gemittelte Reizantwort (vgl. P. A. Tass: Transmission of stimulus-locked responses in two coupled phase oscillators. Phys. Rev. E 69, 051909-1-24 (2004)). Eine Phasenrücksetzung kann festgestellt werden, wenn eine gemittelte Reizantwort detektiert werden kann, d. h., wenn sich im post-Stimulus-Bereich, d. h. im Bereich für t > 0, wobei t = 0 den Anfangszeitpunkt des jeweiligen Reizes darstellt, eine von Null verschiedene Reizantwort findet. Dies kann durch visuelle Inspektion ermittelt werden. Man kann dies auch von der Vorrichtung 2, insbesondere der Steuereinheit 10, durchführen lassen, indem man die prä-Stimulus-Verteilung von $\bar{x}_j(t)$ oder $|\bar{x}_j(t)|$ betrachtet und einen charakteristischen Schwellwert, z. B. die 99te Perzentile der prä-Stimulus-Verteilung von $|\bar{x}_j(t)|$ oder schlicht deren Maximum bestimmt. Wenn nun z. B. der Betrag der post-Stimulus-Antwort prinzipiell oder für eine vorgegebene Mindestdauer, z. B. 20 ms, diesen charakteristischen Schwellenwert übersteigt, liegt eine von Null verschiedene gemittelte Antwort vor. In diesem Fall kann eine Phasenrücksetzung vorliegen. D. h., die Reizstärke müsste so lange erhöht werden, bis die post-Stimulus-Antwort sich von einer Nulllinie unterscheidet. Neben dem hier vorgestellten einfachen, aber in der Praxis bewährten Verfahren können auch andere, dem Fachmann bekannte statistische Tests zur Signalanalyse herangezogen werden.

Eine genauere, aber aufwändigere Variante zur Untersuchung, ob die Reize eine Phasenrücksetzung bewirken, bietet die Analyse der Phase. Hierzu wird die Phase $\psi_j(t)$ von $x_j(t)$ bestimmt. Dies erfolgt mittels Hilbert-Transformation aus dem mittels Bandpassfilterung bzw. empirical mode decomposition bestimmten Signal, welches die pathologische oszillatorische Aktivität repräsentiert. Die empirical mode decomposition ermöglicht im Vergleich zur Bandpassfilterung eine parameterunabhängige Bestimmung physiologisch relevanter Moden in verschiedenen Frequenzbereichen (vgl. N. E. Huang et al.: The empirical mode decomposition and the Hilbert spectrum for nonlinear and non-stationary time series analysis. Proc. R. Soc. A: Math. Phys. Eng. Sci. 454:903-995 (1998)). Die Kombination von empirical mode decomposition mit nachfolgender Hilbert-Transformation wird als Hilbert-Huang-Transformation bezeichnet (vgl. N. E. Huang et al.: A confidence limit for the empirical mode decomposition and Hilbert spectral analysis, Proceedings of the Royal Society of London Series A, 459, 2317-2345 (2003)). Die Phase $\psi_j(t)$ kann auch mittels Wavelet-Analyse bestimmt werden.

Eine Phasenrücksetzung liegt vor, wenn die Phase $\psi_j(t)$ durch einen Reiz (mit Reiz-Beginn bei t = 0) nach einer bestimmten Zeit auf einen Vorzugswert gesetzt wird. D. h., $\{\psi_j(\tau_k + t)\}_{k=1,...,l}$, die von den I Reizantworten gewonnene Verteilung der Werte der Phase $\psi_j(t)$ hat zur Zeit t (relativ zum Burst-Beginn bei t = 0) einen Häufungswert. Dem Fachmann sind unterschiedliche Methoden bekannt, mit denen sich nachweisen lässt, dass eine Verteilung einen Häufungswert (also einen Peak) hat. Eine gebräuchliche Methode ist die Bestimmung des Phasenrücksetzungsindex $\rho(t)$ mittels zirkulärem Mittelwert:

$$\rho(t) = \left| \frac{1}{l}\sum_{k=1}^{l} \exp\left[ i\psi_j(\tau_k + t) \right] \right| \tag{2}$$

Eine Phasenrücksetzung liegt vor, wenn $\rho(t)$ z. B. das Maximum oder die 99te Perzentile der prä-Stimulus-Verteilung von $\rho(t)$ (an einem Zeitpunkt oder innerhalb eines kleinen Zeitfensters von z. B. 20 ms Breite) überschreitet.

In der Praxis hat sich die Analyse mit den gemittelten Antworten $\bar{x}_j(t)$ als ausreichend bewährt.

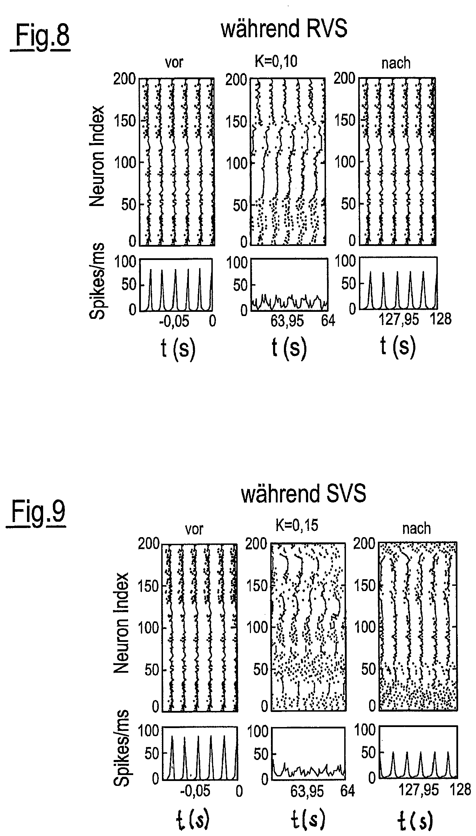

In den Fig. 8 bis 11 werden die mit der hierin beschriebenen Erfindung erzielbaren Effekte anhand von Simulationsergebnissen veranschaulicht. Der Simulation liegt ein Netzwerk von 200 Neuronen zugrunde, wobei alle Neuronen untereinander eine starke anregende kurzreichweitige Kopplung und eine schwache inhibitorische langreichweitige Kopplung aufweisen. Die synaptischen Kopplungsstärken in dem Netzwerk können sich gemäß STDP (Spike Timing-Dependent Plasticity)-Regeln ändern. Ein anfänglich stark gekoppeltes Netzwerk produziert hoch synchrone neuronale Aktivität.

Für die in den Fig. 8 und 9 gezeigten Simulationen wurde die jeweilige CR-Stimulation bei t = 0 s gestartet und bei t = 64 s beendet. Die Aktivität des Netzwerks wurde bis t = 128 s untersucht, d. h., bis 64 s nach Ende der Stimulation. Während der CR-Stimulation wurde kontinuierlich, d. h. ohne Zyklen, in denen Pausen eingehalten wurden, stimuliert.

In den Fig. 8 und 9 zeigen die Abbildungen in der oberen Reihe jeweils, wann jedes einzelne Neuron während verschiedener Zeitintervalle feuert: vor, während und nach Anwendung der CR-Stimulation. Die linke Spalte zeigt die neuronale Aktivität während der letzten 80 ms vor Beginn der CR-Stimulation. Die mittlere Spalte zeigt die neuronale Aktivität während der letzten 80 ms der CR-Stimulation, und die rechte Spalte bezieht sich auf die letzten 80 ms am Ende des anschließenden 64 s dauernden stimulationsfreien Zeitraums. Die Abbildungen in der oberen Reihe sind Rasterplots der neuronalen Aktivität, d. h., elektrische Entladungen der einzelnen Neuronen sind als Punkte gekennzeichnet. Die Abbildungen in der unteren Reihe zeigen jeweils, wie viele Neuronen gleichzeitig innerhalb von 1 ms während der jeweiligen Zeiträume feuern.

Für die in Fig. 8 gezeigte Simulation wurde eine CR-Stimulation mit rasch variierender Sequenz und unterschwelliger Reizstärke (RVS) verwendet. Als Reizstärke wurde K = 0,10 gewählt. Für die in Fig. 9 gezeigte Simulation wurde eine CR-Stimulation mit langsam variierender Sequenz und überschwelliger Reizstärke (SVS) verwendet, wobei die Reizstärke K = 0,15 betrug und jede Sequenz 100mal wiederholt wurde, bevor eine neue Sequenz appliziert wurde. Das Netzwerk befand sich vor der Anwendung der CR-Stimulationen in Fig. 8 und 9 im gleichen Anfangszustand.

Wie die mittlere Spalte von Fig. 8 zeigt, reicht die rasch variierende CR-Stimulation mit geringer Reizstärke für einen Akuteffekt aus, bei dem sich der Synchronisationsgrad während der CR-Stimulation verringert und nach der Beendigung der CR-Stimulation wieder signifikant erhöht. Allerdings führt auch die in Fig. 9 gezeigte alleinige Anwendung einer langsam variierenden CR-Stimulation mit höherer Reizstärke zu keiner langfristigen Desynchronisation.

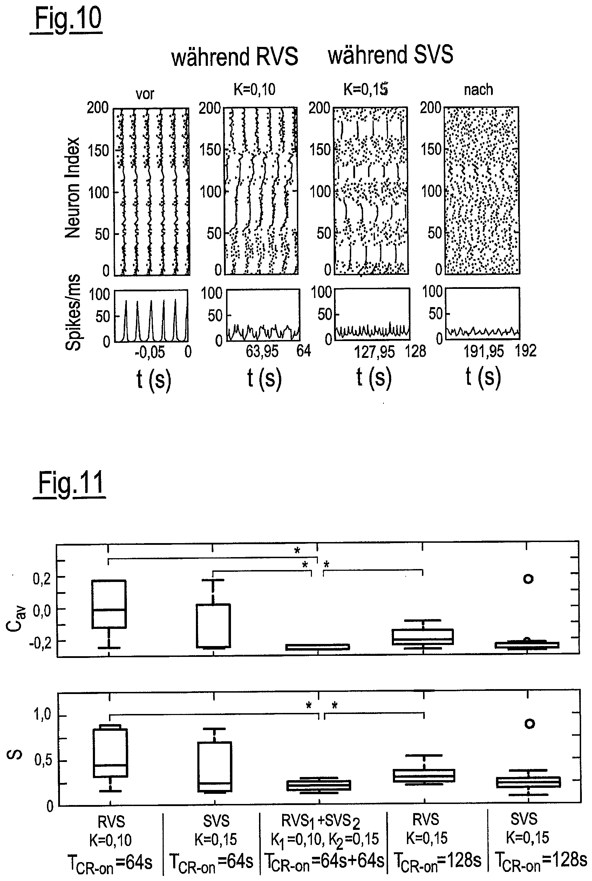

Für die in Fig. 10 gezeigte Simulation wurden die CR-Stimulationen aus den Fig. 8 und 9 miteinander kombiniert. In Fig. 10 zeigt die erste Spalte von links die neuronale Aktivität während der letzten 80 ms vor Beginn der CR-Stimulation. Die zweite Spalte zeigt die neuronale Aktivität während der letzten 80 ms der CR-Stimulation mit rasch variierender Sequenz und unterschwelliger Reizstärke. Die dritte Spalte zeigt die neuronale Aktivität während der letzten 80 ms der CR-Stimulation mit langsam variierender Sequenz und überschwelliger Reizstärke. Die vierte Spalte bezieht sich auf die letzten 80 ms am Ende des anschließenden 64 s dauernden stimulationsfreien Zeitraums. Es wurden dieselben Stimulationsparameter wie in den Fig. 8 und 9 verwendet.

Überraschenderweise zeigt sich, dass durch die nacheinander angewandten CR-Stimulationen mit rasch variierender Sequenz und mit langsam variierender Sequenz ein Stimulationserfolg erzielt wird, der auch nach 64 s nach dem Ende der CR-Stimulation noch anhält.

Da die Anfangsbedingungen des Netzwerks und die Reihenfolgen der aufeinander folgenden Sequenzen von Einfluss auf die Desynchronisation sind, wurden die Simulationen für zehn andere Kombinationen von Anfangsbedingungen des Netzwerks und Sequenzfolgen wiederholt. In Fig. 11 werden die Verteilungen $C_{av}$ der resultierenden Mittelwerte der synaptischen Stärke und die Verteilungen S der Synchronisation nach dem Beenden der CR-Stimulation bei einer Stimulationsdauer von 64 s (zwei linke Spalten) oder 128 s (drei rechte Spalten) als Boxplots für fünf verschiedene Kombinationen von CR-Methoden und Reizstärken K gezeigt. Die totale Dauer der CR-Stimulation wird als $T_{CR-on}$ bezeichnet. Die Mediane der resultierenden $C_{av}$- und S-Verteilungen werden durch die horizontalen Linien in den Boxen (Kästen) dargestellt. Die Box entspricht dem Bereich, in dem die mittleren 50% der Daten liegen, die Antennen ("whiskers") entsprechen den unteren bzw. oberen 25% der Daten. Offene Kreise bezeichnen Ausreißer und sind definiert als Werte, die sich um mindestens das 1,5-fache der Länge der Box unterhalb oder oberhalb der Box befinden. Ein Stern zeigt an, dass die Zwei-Stufen-Stimulation (mittlere Spalte) mit einer anfänglichen CR-Stimulation mit rasch variierender Sequenz und unterschwelliger Reizstärke und einer sich daran anschließenden CR-Stimulation mit langsam variierender Sequenz und überschwelliger Reizstärke statistisch signifikant erfolgreicher ist als die 64 s bzw. 128 s dauernde Ein-Stufen-Stimulation (einseitiger Mann-Whitney-U-Test, p < 0,05).

**Patentansprüche**

1. Vorrichtung (1; 2) zur Stimulation von Neuronen (31), umfassend

    - eine nicht-invasive Stimulationseinheit (11) zur Erzeugung von Reizen (22) in einer Mehrzahl von Stimulationskanälen (12-15), wobei die Stimulationseinheit (11) derart ausgelegt ist, dass die Reize (22) eine Neuronenpopulation (31) im Gehirn und/oder Rückenmark (30) eines Patienten über die Stimulationskanäle an jeweils unterschiedlichen Stellen stimulieren, und
    - eine Steuereinheit (10), welche die Stimulationseinheit (11) während eines ersten Zeitintervalls und eines dem ersten Zeitintervall nachfolgenden zweiten Zeitintervalls in unterschiedlichen Stimulationsmodi betreibt, wobei

- die Steuereinheit (10) die Stimulationseinheit (11) während mindestens 75% der Dauer des ersten Zeitintervalls in einem ersten Stimulationsmodus derart ansteuert, dass die Stimulationseinheit (11) Sequenzen von Reizen (22) repetitiv erzeugt und die Reihenfolge der Stimulationskanäle (12-15), in denen die Reize (22) innerhalb einer Sequenz erzeugt werden, für höchstens 5 nacheinander generierte Sequenzen konstant ist und danach variiert wird,

- die Steuereinheit (10) die Stimulationseinheit (11) während mindestens 75% der Dauer des zweiten Zeitintervalls in einem zweiten Stimulationsmodus derart ansteuert, dass die Stimulationseinheit (11) Sequenzen von Reizen (22) repetitiv erzeugt und die Reihenfolge der Stimulationskanäle (12-15), in denen die Reize (22) innerhalb einer Sequenz erzeugt werden, für mindestens 25 nacheinander generierte Sequenzen konstant ist und danach variiert wird, und

- wobei die Stärke der Reize (22) in dem ersten Stimulationsmodus kleiner oder gleich einer vorgegebenen Reizstärke ist und die Stärke der Reize (22) in dem zweiten Stimulationsmodus mindestens das 1,3-fache der vorgegebenen Reizstärke beträgt.

2. Vorrichtung (1; 2) nach Anspruch 1, wobei die Sequenzen im ersten und/oder zweiten Stimulationsmodus in einem Zeitraster, das aus aufeinanderfolgenden Zyklen besteht, erzeugt werden und zumindest in einigen der Zyklen jeweils eine Sequenz von Reizen (22) erzeugt wird.

3. Vorrichtung (1; 2) nach Anspruch 2, wobei innerhalb eines jeweiligen Zyklus entweder genau eine Sequenz von Reizen (22) erzeugt wird oder keine Reize erzeugt werden.

4. Vorrichtung (1; 2) nach Anspruch 2 oder 3, wobei während n aufeinanderfolgenden Zyklen Reize (22) erzeugt werden und während der darauffolgenden m Zyklen keine Reize erzeugt werden und dieses Muster periodisch fortgesetzt wird, wobei n und m nicht-negative ganze Zahlen sind.

5. Vorrichtung (1; 2) nach einem der vorhergehenden Ansprüche, wobei das Muster, nach welchem im ersten Stimulationsmodus die Reihenfolge der Stimulationskanäle (12-15), in denen die Reize (22) innerhalb einer Sequenz erzeugt werden, für höchstens 5 nacheinander generierte Sequenzen konstant ist und danach die Reihenfolge variiert wird, mehrfach wiederholt wird.

6. Vorrichtung (1; 2) nach einem der vorhergehenden Ansprüche, wobei das Muster, nach welchem im zweiten Stimulationsmodus die Reihenfolge der Stimulationskanäle (12-15), in denen die Reize (22) innerhalb einer Sequenz erzeugt werden, für mindestens 25 nacheinander generierte Sequenzen konstant ist und danach die Reihenfolge variiert wird, mehrfach wiederholt wird.

7. Vorrichtung (1; 2) nach einem der vorhergehenden Ansprüche, wobei die Stimulationseinheit (11) innerhalb einer jeweiligen Sequenz in jedem Stimulationskanal (11-15) genau einen Reiz (22) erzeugt.

8. Vorrichtung (1; 2) nach Anspruch 7, wobei genau ein Reiz (22) genau ein Pulszug ist.

9. Vorrichtung (2) nach einem der vorhergehenden Ansprüche, umfassend eine Messeinheit (16) zum Aufnehmen von Messsignalen (23), die eine neuronale Aktivität der mit den Reizen (22) stimulierten Neuronenpopulation (31) wiedergeben.

10. Vorrichtung (2) nach Anspruch 9, wobei die Steuereinheit (10) derart ausgelegt ist, dass sie von dem ersten Stimulationsmodus in den zweiten Stimulationsmodus umschaltet, wenn die Steuereinheit (10) anhand der Messsignale (23) feststellt, dass ein Synchronisationsgrad der stimulierten Neuronenpopulation (31) durch die Applikation der Reize (22) im ersten Stimulationsmodus um mindestens einen vorgegebenen Schwellwert reduziert wird.

11. Vorrichtung (1; 2) nach einem der vorhergehenden Ansprüche, wobei die Reize (22) akustische, visuelle, taktile, vibratorische, thermische, olfaktorische, gustatorische, transkutane elektrische, transkutane magnetische, transkranielle elektrische und/oder transkranielle magnetische Reize und/oder Ultraschall-Reize sind.

12. Computerprogrammprodukt umfassend Programmteile, welche, wenn in einem Computer geladen, zur Durchführung eines computer-implementierten Verfahrens ausgelegt sind, bei welchem

- eine nicht-invasive Stimulationseinheit (11) Reize (22) in einer Mehrzahl von Stimulationskanälen (12-15) erzeugt, wobei die Reize (22) eine Neuronenpopulation (31) im Gehirn und/oder Rückenmark (30) eines Patienten über die Stimulationskanäle (12-15) an jeweils unterschiedlichen Stellen stimulieren, wobei

- die Stimulationseinheit (11)-während eines ersten Zeitintervalls und eines dem ersten Zeitintervall nachfolgenden zweiten Zeitintervalls in unterschiedlichen Stimulationsmodi betrieben wird,
- die Stimulationseinheit (11) während mindestens 75% der Dauer des ersten Zeitintervalls in einem ersten Stimulationsmodus derart betrieben wird, dass die Stimulationseinheit (11) Sequenzen von Reizen (22) repetitiv erzeugt und die Reihenfolge der Stimulationskanäle (12-15), in denen die Reize (22) innerhalb einer Sequenz erzeugt werden, für höchstens 5 nacheinander generierte Sequenzen konstant ist und danach variiert wird,
- die Stimulationseinheit (11) während mindestens 75% der Dauer des zweiten Zeitintervalls in einem zweiten Stimulationsmodus derart betrieben wird, dass die Stimulationseinheit (11) Sequenzen von Reizen (22) repetitiv erzeugt und die Reihenfolge der Stimulationskanäle (12-15), in denen die Reize (22) innerhalb einer Sequenz erzeugt werden, für mindestens 25 nacheinander generierte Sequenzen konstant ist und danach variiert wird, und
- wobei die Stärke der Reize (22) in dem ersten Stimulationsmodus kleiner oder gleich einer vorgegebenen Reizstärke ist und die Stärke der Reize (22) in dem zweiten Stimulationsmodus mindestens das 1,3-fache der vorgegebenen Reizstärke beträgt.

## Claims

1.  A device (1; 2) for stimulating neurons (31), comprising

    - a non-invasive stimulation unit (11) for generating stimuli (22) in a plurality of stimulation channels (12-15), the stimulation unit (11) being designed such that the stimuli (22) stimulate a neuron population (31) in the brain and/or spinal cord (30) of a patient via the stimulation channels at respectively different points, and
    - a control unit (10), which operates the stimulation unit (11) during a first time interval and a second time interval following the first time interval in different stimulation modes, wherein
    - the control unit (10) controls the stimulation unit (11) during at least 75% of the duration of the first time interval in a first stimulation mode such that the stimulation unit (11) repetitively generates sequences of stimuli (22) and the order of the stimulation channels (12-15), in which the stimuli (22) are generated within a sequence, is constant for not more than 5 successively generated sequences and is then varied,
    - the control unit (10) controls the stimulation unit (11) during at least 75% of the duration of the second time interval in a second stimulation mode such that the stimulation unit (11) repetitively generates sequences of stimuli (22) and the order of the stimulation channels (12-15), in which the stimuli (22) are generated within a sequence, is constant for at least 25 successively generated sequences and is then varied, and
    - wherein the intensity of the stimuli (22) in the first stimulation mode is lower than or equal to a predetermined stimulus intensity and the intensity of the stimuli (22) in the second stimulation mode is at least 1.3 times the predetermined stimulus intensity.

2.  The device (1; 2) according to claim 1, wherein the sequences in the first and/or second stimulation mode are generated in a time pattern consisting of consecutive cycles, and at least in some of the cycles a sequence of stimuli (22) is generated respectively.

3.  The device (1; 2) according to claim 2, wherein, within a respective cycle, either exactly one sequence of stimuli (22) is generated or no stimuli are generated.

4.  The device (1; 2) according to claim 2 or 3, wherein stimuli (22) are generated during n consecutive cycles (22) and no stimuli are generated during the subsequent m cycles, and this pattern is periodically continued, wherein n and m are non-negative integers.

5.  The device (1; 2) according to one of the preceding claims, wherein the pattern according to which, in the first stimulation mode, the order of the stimulation channels (12-15), in which the stimuli (22) are generated within a sequence, is constant for not more than 5 successively generated sequences and the order is then varied, is repeated several times.

6.  The device (1; 2) according to one of the preceding claims, wherein the pattern according to which, in the second stimulation mode, the order of the stimulation channels (12-15), in which the stimuli (22) are generated within a sequence, is constant for at least 25 successively generated sequences and the order is then varied, is repeated several times.

7. The device (1; 2) according to one of the preceding claims, wherein the stimulation unit (11) generates precisely one stimulus (22) within a respective sequence in each stimulation channel (11-15).

8. The device (1; 2) according to claim 7, wherein precisely one stimulus (22) is exactly one pulse train.

9. The device (2) according to one of the preceding claims, comprising a measuring unit (16) for recording measurement signals (23) representing a neuronal activity of the neuron population (31) stimulated with the stimuli (22).

10. The device (2) according to claim 9, wherein the control unit (10) is designed such that it switches from the first stimulation mode to the second stimulation mode when the control unit (10) determines, based on the measurement signals (23), that a degree of synchronization of the stimulated neuron population (31) is reduced by at least one predetermined threshold value by application of the stimuli (22) in the first stimulation mode.

11. The device (1; 2) according to one of the preceding claims, wherein the stimuli (22) are acoustic, visual, tactile, vibratory, thermal, olfactory, gustatoric, transcutaneous electrical, transcutaneous magnetic, transcranial electrical and/or transcranial magnetic stimuli and/or ultrasonic stimuli.

12. A computer program product comprising program parts, which, when loaded in a computer, are adapted to execute a computer-implemented method in which

- a non-invasive stimulation unit (11) generates stimuli (22) in a plurality of stimulation channels (12-15), wherein the stimuli (22) stimulate a neuron population (31) in the brain and/or spinal cord (30) of a patient via the stimulation channels (12-15) at respectively different points,
- the stimulation unit (11) is operated in different stimulation modes during a first time interval and a second time interval following the first time interval,
- the stimulation unit (11) is operated in a first stimulation mode during at least 75% of the duration of the first time interval such that the stimulation unit (11) repetitively generates sequences of stimuli (22) and the order of the stimulation channels (12-15), in which the stimuli (22) are generated within a sequence, is constant for not more than 5 successively generated sequences and is then varied,
- the stimulation unit (11) is operated in a second stimulation mode during at least 75% of the duration of the second time interval such that the stimulation unit (11) repetitively generates sequences of stimuli (22) and the order of the stimulation channels (12-15), in which the stimuli (22) are generated within a sequence, is constant for at least 25 successively generated sequences and is then varied, and
- wherein the intensity of the stimuli (22) in the first stimulation mode is lower than or equal to a predetermined stimulus intensity and the intensity of the stimuli (22) in the second stimulation mode is at least 1.3 times the predetermined stimulus intensity.

**Revendications**

1. Dispositif (1 ; 2) de stimulation de neurones (31) comprenant

- un module de stimulation non invasif (11) pour la génération de stimuli (22) dans une pluralité de canaux de stimulation (12 à 15), dans lequel le module de stimulation (11) est conçu de telle sorte que les stimuli (22) stimulent une population de neurones (31) dans le cerveau et/ou la moelle épinière (30) d'un patient par le biais des canaux de stimulation à différents endroits respectivement, et
- un module de commande (10) qui exploite le module de stimulation (11) pendant un premier intervalle de temps et un second intervalle de temps succédant au premier intervalle de temps dans différents modes de stimulation, dans lequel
- le module de commande (10) commande le module de stimulation (11) pendant au moins 75 % de la durée du premier intervalle de temps dans un premier mode de stimulation de telle sorte que le module de stimulation (11) génère des séquences de stimuli (22) de manière répétitive et l'ordre des canaux de stimulation (12 à 15) dans lesquels les stimuli (22) sont générés au sein d'une séquence est constant pour au plus 5 séquences générées successivement et est ensuite varié,
- le module de commande (10) commande le module de stimulation (11) pendant au moins 75 % de la durée du second intervalle de temps dans un second mode de stimulation de telle sorte que le module de stimulation (11) génère des séquences de stimuli (22) de manière répétitive et l'ordre des canaux de stimulation (12 à 15) dans lesquels les stimuli (22) sont générés au sein d'une séquence est constant pour au moins 25 séquences générées successivement et est ensuite varié, et

- dans lequel l'intensité des stimuli (22) dans le premier mode de stimulation est inférieure ou égale à une intensité de stimulus prédéfinie et l'intensité des stimuli (22) dans le second mode de stimulation est au moins 1,3 fois l'intensité de stimulus prédéfinie.

2. Dispositif (1 ; 2) selon la revendication 1, dans lequel les séquences dans le premier et/ou second mode de stimulation sont générées dans un schéma de temps qui se compose de cycles successifs et une séquence de stimuli (22) est à chaque fois générée au moins dans certains des cycles.

3. Dispositif (1 ; 2) selon la revendication 2, dans lequel soit exactement une séquence de stimuli (22) est générée, soit aucun stimulus n'est généré au sein d'un cycle respectif.

4. Dispositif (1 ; 2) selon la revendication 2 ou 3, dans lequel des stimuli (22) sont générés pendant n cycles successifs et aucun stimulus n'est généré pendant les m cycles subséquents et ce modèle est poursuivi périodiquement, dans lequel n et m sont des nombres entiers non négatifs.

5. Dispositif (1 ; 2) selon une des revendications précédentes, dans lequel le modèle, d'après lequel l'ordre des canaux de stimulation (12 à 15) dans le premier mode de stimulation dans lesquels les stimuli (22) sont générés au sein d'une séquence est constant pour au plus 5 séquences générées successivement et l'ordre est ensuite varié, est répété plusieurs fois.

6. Dispositif (1 ; 2) selon une des revendications précédentes, dans lequel le modèle, d'après lequel l'ordre des canaux de stimulation (12 à 15) dans le second mode de stimulation dans lesquels les stimuli (22) sont générés au sein d'une séquence est constant pour au moins 25 séquences générées successivement et l'ordre est ensuite varié, est répété plusieurs fois.

7. Dispositif (1 ; 2) selon une des revendications précédentes, dans lequel le module de stimulation (11) génère exactement un stimulus (22) au sein d'une séquence respective dans chaque canal de stimulation (11 à 15).

8. Dispositif (1 ; 2) selon la revendication 7, dans lequel exactement un stimulus (22) est exactement un train d'impulsions.

9. Dispositif (2) selon une des revendications précédentes, comprenant un module de mesure (16) pour l'enregistrement de signaux de mesure (23) qui restituent une activité neuronale de la population de neurones (31) stimulée avec les stimuli (22).

10. Dispositif (2) selon la revendication 9, dans lequel le module de commande (10) est conçu de telle sorte qu'il commute du premier mode de stimulation au second mode de stimulation lorsque le module de commande (10) constate à l'aide des signaux de mesure (23) qu'un degré de synchronisation de la population de neurones stimulée (31) est réduit d'au moins une valeur seuil prédéfinie par l'application des stimuli (22) dans le premier mode de stimulation.

11. Dispositif (1 ; 2) selon une des revendications précédentes, dans lequel les stimuli (22) sont des stimuli acoustiques, visuels, tactiles, vibratoires, thermiques, olfactifs, gustatifs, électriques transcutanés, magnétiques transcutanés, électriques transcrâniens et/ou magnétiques transcrâniens et/ou des stimuli ultrasoniques.

12. Produit de programme informatique comprenant des parties de programme qui, lorsqu'elles sont chargées dans un ordinateur, sont conçues pour la réalisation d'un procédé mis en oeuvre par ordinateur, lors duquel

- un module de stimulation non invasif (11) génère des stimuli (22) dans une pluralité de canaux de stimulation (12 à 15), dans lequel les stimuli (22) stimulent une population de neurones (31) dans le cerveau et/ou la moelle épinière (30) d'un patient par le biais des canaux de stimulation (12 à 15) à différents endroits respectivement, dans lequel
- le module de stimulation (11) est exploité pendant un premier intervalle de temps et un second intervalle de temps succédant au premier intervalle de temps dans différents modes de stimulation,
- le module de stimulation (11) est exploité pendant au moins 75 % de la durée du premier intervalle de temps dans un premier mode de stimulation de telle sorte que le module de stimulation (11) génère des séquences de stimuli (22) de manière répétitive et l'ordre des canaux de stimulation (12 à 15) dans lesquels les stimuli (22) sont générés au sein d'une séquence est constant pour au plus 5 séquences générées successivement et est ensuite varié,

- le module de stimulation (11) est exploité pendant au moins 75 % de la durée du second intervalle de temps dans un second mode de stimulation de telle sorte que le module de stimulation (11) génère des séquences de stimuli (22) de manière répétitive et l'ordre des canaux de stimulation (12 à 15) dans lesquels les stimuli (22) sont générés au sein d'une séquence est constant pour au moins 25 séquences générées successivement et est ensuite varié, et

- dans lequel l'intensité des stimuli (22) dans le premier mode de stimulation est inférieure ou égale à une intensité de stimulus prédéfinie et l'intensité des stimuli (22) dans le second mode de stimulation est au moins 1,3 fois l'intensité de stimulus prédéfinie.

## Fig.1

## Fig.2

## Fig.3A

## Fig.3B

Fig.4

Fig.5

## Fig.6

## Fig.7

Fig.8

während RVS

Fig.9

während SVS

## Fig.10

während RVS     während SVS

## Fig.11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102010016404 A1

**In der Beschreibung aufgeführte Nicht-Patentliteratur**

- **P. A. TASS ; I. ADAMCHIC ; H.-J. FREUND ; T. VON STACKELBERG ; C. HAUPTMANN.** Counteracting tinnitus by acoustic coordinated reset neuromodulation. *Restorative Neurology and Neuroscience,* 2012, vol. 30, 137-159
- **I. ADAMCHIC ; T. TOTH ; C. HAUPTMANN ; P. A. TASS.** Reversing pathologically increased EEG power by acoustic CR neuromodulation. *Human Brain Mapping,* 2014, vol. 35, 2099-2118
- **A. N. SILCHENKO ; I. ADAMCHIC ; C. HAUPTMANN ; P. A. TASS.** Impact of acoustic coordinated reset neuromodulation on effective connectivity in a neural network of phantom sound. *Neuroimage,* 2013, vol. 77, 133-147

- **I. ADAMCHIC ; B. LANGGUTH ; C. HAUPTMANN ; P. A. TASS.** Abnormal brain activity and cross-frequency coupling in the tinnitus network. *Frontiers in Neuroscience,* 2014, vol. 8, 284
- **P. A. TASS.** Transmission of stimulus-locked responses in two coupled phase oscillators. *Phys. Rev.,* 2004, vol. E 69, 051909-1, 24
- **N. E. HUANG et al.** The empirical mode decomposition and the Hilbert spectrum for nonlinear and non-stationary time series analysis. *Proc. R. Soc. A: Math. Phys. Eng. Sci.,* 1998, vol. 454, 903-995
- **N. E. HUANG et al.** A confidence limit for the empirical mode decomposition and Hilbert spectral analysis. *Proceedings of the Royal Society of London Series A,* 2003, vol. 459, 2317-2345